# EUROPEAN PATENT SPECIFICATION

(11) **EP 3 806 738 B1**
(45) Date of publication and mention of the grant of the patent: **24.04.2024**
(21) Application number: 19731692.0
(22) Date of filing: 14.06.2019
(51) Int. Cl.: A61B 5/11, A61B 5/00

(54) **DEVICE HOUSING AND MOUNTING IN USER ACTIVITY MONITORING SYSTEMS**
GERÄTEGEHÄUSE UND HALTERUNG IN SYSTEMEN ZUR ÜBERWACHUNG VON BENUTZERAKTIVITÄTEN
BOÎTIER ET MONTAGE DE DISPOSITIF DANS DES SYSTÈMES DE SURVEILLANCE D'ACTIVITÉ D'UTILISATEUR

(30) Priority: 14.06.2018 GB 201809756; 28.08.2018 GB 201813969; 01.03.2019 GB 201902774
(43) Date of publication of application: 21.04.2021
(73) Proprietor: T.J.SMITH AND NEPHEW, LIMITED, Hull HU3 2BN (GB)
(72) Inventor: CAUWOOD, Peter David, Cambridge CB4 3DN (GB); DAGEVOS VAN RIJ, Johannes, Hull HU3 2BN (GB); DOWLER, Allan, David, Hull HU3 2BN (GB); GOWANS, Philip, Hull HU3 2BN (GB); HARTWELL, Edward Yerbury, Hull HU3 2BN (GB); HUNT, Allan Kenneth Frazer Grugeon, Hull HU3 2BN (GB); LAITENBERGER, Peter Georg, Cambridge CB4 3DN (GB); PARTINGTON, Lee Ian, Hull HU3 2BN (GB); PHILLIPS, Marcus Damian, Hull HU3 2BN (GB); QUINTANAR, Felix Clarence, Hull HU3 2BN (GB); SABBERTON, Iain James, Cambridge CB4 3DN (GB); SMITH, Damian Lawson, Hull HU3 2BN (GB); STRACHAN, Kirsty Margaret, Hull HU3 2BN (GB); URWIN, Charlotte, Hull HU3 2BN (GB); WARREN-BARRATT, Emily Grace, Hull HU3 2BN (GB)
(74) Representative: Smith & Nephew
(86) International application number: PCT/EP2019/065684
(87) International publication number: WO 2019/238927

(56) References cited:
- US-A1- 2003 163 287
- US-A1- 2009 292 194
- US-A1- 2014 203 797
- US-A1- 2014 206 976
- US-A1- 2017 281 073

## Description

### CROSS-REFERENCE TO RELATED APPLICATIONS

This application claims priority to U.K. Provisional Application Nos. 1809756.8, 1813969.1, and 1902774.7 respectively filed on June 14, 2018, August 28, 2018, and March 1, 2019.

### BACKGROUND

### Field

Embodiments of the present disclosure relate to devices for the monitoring of body loading and body position for treatment of pressure ulcers.

### Description of the Related Art

Pressure ulcers, which are also known as pressure sores, bedsores, or decubitus ulcers, are injuries to skin and underlying tissue resulting from prolonged pressure on the skin, soft tissue, muscle, or bone above capillary filling pressure (approximately 32 mmHg). Pressure ulcers may typically develop on skin that covers bony areas, such as heels, ankles, hips, shoulder blades, spine, elbows, back of the head, and tailbone. Persons whose mobility is limited due to age or medical conditions are at an increased risk of developing pressure ulcers because of their inability to change positions while sitting or lying down. Management and treatment of pressure ulcers include repositioning of the injured limb or body part and using support surfaces, such as a mattress, cushion, or the like.

One type of pressure ulcer that develops on a foot is known as a diabetic foot ulcer (DFU), which tends to occur with higher frequency and intensity in the diabetic population. Management and treatment of diabetic foot ulcers requires offloading the wound by using cushioned footwear, such as a support boot, cast, shoe, etc. While offloading can be effective, because many offloading devices are removable, it has been found that patient non-compliance with the offloading devices plays a large role in the delayed healing of diabetic foot ulcers.

However, prior art approaches and systems provide no or little information regarding patients' lifestyle and non-compliance or compliance with the offloading devices and support surfaces. Gaining insight into patients' lifestyle can be important for prevention and healing of pressure ulcers. However, because of these limitations, prevention and healing of patients' pressure ulcers using prior art approaches and systems may be delayed or, worse yet, the condition could worsen leading to prolonged discomfort, hospitalization, or even surgery.

The invention is defined in claim 1.

### BRIEF DESCRIPTION OF THE DRAWINGS

Embodiments of the present disclosure will now be described hereinafter, by way of example only, with reference to the accompanying drawings in which:
Figure 1 illustrates a user activity monitoring system including an example activity monitoring device;
Figure 2 illustrates example components of the activity monitoring device of Figure 1 and a data processing device;
Figure 3A illustrates an embodiment of a housing ofthe activity monitoring device of Figure 1;
Figure 3B illustrates the activity monitoring device of Figure 3A;
Figure 4A illustrates an embodiment of a housing ofthe activity monitoring device of Figure 1;
Figure 4B illustrates the activity monitoring device of Figure 4A;
Figure 5A illustrates an embodiment of a housing ofthe activity monitoring device of Figure 1;
Figure 5B illustrates the activity monitoring device of Figure 5A;
Figure 6A illustrates an embodiment of a housing ofthe activity monitoring device of Figure 1;
Figure 6B illustrates the activity monitoring device of Figure 6A;
Figure 7A illustrates an embodiment of a housing ofthe activity monitoring device of Figure 1;
Figure 7B illustrates the activity monitoring device of Figure 7A;
Figure 8A illustrates an embodiment of a housing ofthe activity monitoring device of Figure 1;
Figure 8B illustrates the activity monitoring device of Figure 8A;
Figure 9A illustrates an embodiment of a housing of the activity monitoring device of Figure 1;
Figure 9B illustrates the activity monitoring device of Figure 9A;
Figure 10A illustrates an embodiment of a housing of the activity monitoring device of Figure 1;
Figure 10B illustrates the activity monitoring device of Figure 10A;
Figure 11A illustrates an embodiment of a housing of the activity monitoring device of Figure 1;
Figure 11B illustrates the activity monitoring device of Figure 11A;
Figure 12 illustrates an embodiment of a housing of the activity monitoring device of Figure 1;
Figure 13 illustrates a side cross-sectional view of an embodiment of a housing of the activity monitoring device of Figure 1;
Figure 14 illustrates a perspective view of the inner surface of an embodiment of a cap portion of a housing of the activity monitoring device of Figure 1;
Figure 15A illustrates the inside surfaces of the cap portion and base portion of the housing of Figure 14;
Figure 15B illustrates the inside surfaces of the cap portion and base portion of the housing of Figure 14;
Figure 15C illustrates the assembled housing of Figure 14;
Figure 15D illustrates the assembled housing of Figure 14;
Figure 16 illustrates a printed circuit board assembly (PCBA) positioned in a cap portion of a housing of the activity monitoring device of Figure 1;
Figures 17A and 17B illustrate an embodiment of the activity monitoring device of Figure 1 that includes an example dressing;
Figure 18 illustrates an embodiment of a power source and an associated mount;
Figure 19A illustrates an embodiment of a cap portion of a housing of the activity monitoring device of Figure 1 alongside a PCBA;
Figure 19B illustrates the PCBA of Figure 19A aligned for placement in the cap portion of a housing of the activity monitoring device of Figure 19A;
Figure 19C illustrates the PCBA of Figure 19A positioned in the cap portion of a housing of the activity monitoring device of Figure 19A with the power source and the associated mount of Figure 18 connected;
Figures 20A and 20B respectively illustrate side and top views of an embodiment of a base portion of a housing of the activity monitoring device of Figure 1 that includes an example adhesive applied;
Figure 21 illustrates an embodiment of an example wrapping positioned for enclosing a housing of the activity monitoring device of Figure 1;
Figure 22A illustrates an embodiment of an adhesive applied to a base portion of a housing of the activity monitoring device of Figure 1;
Figure 22B illustrates an embodiment of the housing of the activity monitoring device of Figure 22A positioned on an example dressing;
Figure 22C illustrates a positioning of the housing of the activity monitoring device and the dressing of Figure 22A on a curing shelf;
Figure 23A illustrates a top view of a housing of the activity monitoring device of Figure 1 positioned on an example dressing;
Figure 23B illustrates a side view of the housing of the activity monitoring device and the dressing of Figure 23A with components spaced apart from one another;
Figure 23C illustrates an embodiment of the activity monitoring device of Figure 1 that includes an example dressing and components unassembled;
Figure 24A illustrates an embodiment of a cap portion of a housing of the activity monitoring device of Figure 1;
Figure 24B and 24C illustrate side and perspective views of an embodiment of a base portion of a housing of the activity monitoring device of Figure 1;
Figure 25A illustrates an embodiment of the activity monitoring device of Figure 1 that includes an example dressing and components unassembled;
Figure 25B illustrates a side view of a housing of the activity monitoring device and the dressing of Figure 25A;
Figure 25C illustrates a top view of a housing of the activity monitoring device and the dressing of Figure 25A;
Figure 26A illustrates an embodiment of the activity monitoring device of Figure 1 that includes an example dressing and components unassembled;
Figure 26B illustrates a side view of a housing of the activity monitoring device and the dressing of Figure 26A;
Figure 26C illustrates a top view of a housing of the activity monitoring device and the dressing of Figure 26A;
Figure 27A illustrates a side view of an embodiment of the activity monitoring device of Figure 1 with the components of the activity monitoring device spaced apart from one another;
Figure 27B illustrates a top view of a housing of the activity monitoring device and the dressing of Figure 27A;
Figure 28A illustrates a side view of an embodiment of the activity monitoring device of Figure 1 with the components of the activity monitoring device spaced apart from one another;
Figure 28B illustrates an embodiment of a top view of a housing of the activity monitoring device and the dressing of Figure 28A;
Figure 28C illustrates of an alternative embodiment of a top view of a housing of the activity monitoring device and the dressing of Figure 28A;
Figure 29A illustrates of a side view of an embodiment of the activity monitoring device of Figure 1 with the components of the activity monitoring device spaced apart from one another;
Figure 29B illustrates a top view of a housing of the activity monitoring device and the dressing of Figure 29A;
Figure 30A illustrates of a side view of an embodiment of the activity monitoring device of Figure 1 with the components of the activity monitoring device spaced apart from one another;
Figure 30B illustrates a top view of a housing of the activity monitoring device and the dressing of Figure 30A;
Figure 31A illustrates of a side view of an embodiment of the activity monitoring device of Figure 1 with the components of the activity monitoring device spaced apart from one another;
Figure 31B illustrates a top view of a housing of the activity monitoring device and the dressing of Figure 31A;
Figure 32A illustrates of a side view of an embodiment of the activity monitoring device of Figure 1 with the components of the activity monitoring device spaced apart from one another;
Figure 32B illustrates a top view of a housing of the activity monitoring device and the dressing of Figure 32A;
Figure 33A illustrates of a side view of an embodiment of the activity monitoring device of Figure 1 with the components of the activity monitoring device spaced apart from one another;
Figure 33B illustrates a top view of a housing of the activity monitoring device and the dressing of Figure 33A;
Figure 34 illustrates a side view of an embodiment of the activity monitoring device with a housing attached to a dressing; and
Figure 35 illustrates a side view of an embodiment of the activity monitoring device with a housing attached to a dressing.
Figure 36 illustrates an exploded view of an embodiment of a dressing to which the activity monitoring device can be attached.

### DETAILED DESCRIPTION

### Overview

An activity monitoring device can be a wearable component usable for monitoring diabetic foot ulcers. The activity monitoring device can include a sensor or number of sensors, including an accelerometer (such as, a 3 axis accelerometer), gyroscope, magnetometer, barometric pressure (barometer), light (for example, usable for identification of day/night cycles, initiation like removal from opaque packaging or opaque release handle/label, failure of adhesion such as with sensor pointing at tissue and lit up if the activity monitoring device falls off, or data transmission), or temperature. The one or more sensors can be mounted on a single or multi-layer board (for example, FR4 board) or other substrate (for example, polyimide). Multiple types of sensors (or elements within sensors) can be combined to use as an inertial measurement unit (INU) (for example, 6-axis for 2 sensors or 9-axis for 3 sensors), even if one sensor (such as, an accelerometer) may be alone in actively reading (for example, powered up) for some or all of the time. In some implementations, the use of an accelerometer, gyroscope, and magnetometer as a 9-axis INU can be utilized initially to identify datum positions and angles, and then shut down one or more elements or components (such as, power hungry components) for some or all of the time.

The activity monitoring device can be worn just below the knee and may be towards the inside of the knee. The activity monitoring device can be encapsulated or otherwise encased in housing. The activity monitoring device can be attached using an adhesive, a film adhesive, or via a garment. The activity monitoring device or one or more sensors with the activity monitoring device can include a portable energy source, such as a battery. The removal of unrequired sensors can be facilitated in some implementations to make use of the finite energy budget available or minimise the risk of noise from external sources on both signal and software.

The activity monitoring device can be applied to different surfaces for monitoring the different surfaces, and individual activity monitoring devices can thus be used to monitor different parts of a user activity monitoring system. For example, one activity monitoring device can monitor a patient while another activity monitoring device can monitor an offloading device (for example, offloading boots, insoles, crutches, or other mobility aids), such as that may be used by a patient with a foot ulcer (for example, a diabetic foot ulcer). The

A housing of the activity monitoring device can have one or more of the following features. The housing can have a thinned out region to allow the user to press a button within the housing while providing protection to avoid an unintentional operation of the button. This region can be in the form of a triangular cut-out and formed within the top-part of the housing to avoid a separate button. Other parts of the housing can designed to be stiffer than this button area. The area can be shaped and sized to accommodate the pad of a thumb. A wide range of button designs can be used. The thinned out region can also allow a light source to shine through, and the light level shining through can be tailored by selecting the thickness of the housing or by selecting a transparency and color of the housing. There can be on the inside of the housing which allow a circuit board to be positioned or supported. For instance, circumferential constraints or 4 pins at 90 degrees spacing can fix a rotation and lateral alignment of the circuit board with respect to the housing, while a ledge fixes the height of the circuit board with respect to the top surface. This can facilitate, for example, the circuit board being in a correct orientation and at a correct height to provide interaction with the button and also aligns the light source in the thinned out area. Within the top molding, parts of the lid can be deliberately made to be stiff by using a thicker material thickness, and these thicker areas can be cored out in order ensures that the part is moldable, avoid sinkage, and maintain the stiffness of these areas while reducing the overall weight. The bottom molding can have a largely flat surface with a small radius of curvature on the outside to allow the part to be attached to the human body while avoiding clothes to be caught under the edge and ripping the part off. In addition, the bottom surface can have a small recess to allow for the attachment of a double-sided adhesive tape to attach the tracker to a person. The top molding can be shaped in a rounded and smooth manner to avoid any point where the activity monitoring device may catch with clothes or another object and may also to give the activity monitoring device a smaller visual footprint. The housing can be made by stereolithography (SLA) or polyjet from photopolymer 3D printing material with a Shore hardness of 73 - 77 on scale A, or by 3D printing from an engineering resin with a Shore hardness of 80A. The housing can include an elastomer, a thermoplastic elastomer, or be constructed by injection molding. The molded parts can be made from liquid silicone rubber in white. An adhesive (for example, one for attaching plastics and elastomeric materials) can be used to glue the two moldings together, and a wide range of other adhesives (for example, cyanoacrylates, silicones, epoxies, hydrogels, hydrocolloids, sealant systems) or other techniques (for example use of double-sided adhesive tapes, ultrasonic welding, staking etc.) can be used. The top and bottom housings can be designed to have a very small gap between them in the assembled state in order to allow for the adhesive thickness but also to allow for a circuit board to be slightly clamped in the assembled housing (to prevent "rattling" of the circuit board within the housing and unintended button presses). The intention can be that a slight pressure is applied between the top and bottom housings while the adhesive is setting. When assembled, the features in the top and bottom housing can form a convoluted path to assist with ingress protection. Features in the side walls of both moldings can allow the parts to be mechanically held together or grooves for adhesive. Pressure or light sensors or other components can be removed from the activity monitoring device to reduce power consumption.

The housing can be sealed, flexible, and have a known Shore hardness. The housing can have a mechanical structure and design features that provide for a shouldered keyway alignment of elements of the activity monitoring device. The housing can include areas designed for support of the circuit board. The housing can have a flexible surface above the circuit board, and a pin designed to provide force onto a button above the board. The housing can include thin areas for viewing of a light emitting diode (LED) enclosed within the housing. The housing can include case work that touches components of the activity monitoring device (for example, the printed circuit board assembly). Portions of the housing can be made clear for visible or ultraviolet (UV) light and to enhance visualization of the LED encased in the case work of the activity monitoring device when the LED is activated. The case work or housing can have a hard base and a soft top, or a soft base and hard top. The housing can be adapted to seal both sides of the housing with an adhesive material. The housing can be sealed or constructed so that, upon unsealing or deconstruction of the housing (for example, by separation of two portions of the housing) or tampering with the housing, one or more components positioned in the housing are destroyed or cease properly functioning. For instance, one component in the housing (such as a circuit board or a component attached to the circuit board) can be attached to a first portion of the housing and another component in the housing can be attached to a second portion ofthe housing, so upon separation of the first portion and the second portion, the one component and the another component can be separated causing the one component or the another component to cease to function properly (such as by breaking an electrical connection between the one component and the another component or by breaking an electrical on the one component or the another component that breaks a functionality). The housing can be adhered to the patient of an orthopedic device by using an adhesive film. Alternatively a glue such as a super glue for use on tissue could be used to bond the housing to an adhesive film. The adhesive film may be an IV3000 dressing, such as Opsite Flexigrid dressing or any other adhesive film dressing already used in wound care. The adhesive can be a medical or healthcare field grade adhesive. Alternatively the adhesive can be any adhesive film that is tolerable on human skin. The adhesive on the film can be acrylic or silicone gel.

The activity monitoring device can be adhered to, sewn into, placed within etc. clothing, such as a knee brace, a knee strap, a garter, or a snug-fitting tube of fabric that is worn over a portion of a limb. The activity monitoring device could be retained in place by inserting in the device in a pocket at the knee, in a pocket in some suitable garment, including trousers, knee braces etc. The activity monitoring device could be secured to a user or an orthopedic device using a strap - such as similar to a watch strap or belt could be used, or using a clip - for example, similar to a money clip.

The activity monitoring device can be retained in the lacing of a shoe or other garment. The activity monitoring device can be integrated into a shoe. The activity monitoring device can be given a color that promotes discretion or blending in of the activity monitoring device, such as by matching the color of the device to the skin tone. The activity monitoring device can be given a color to allow the product to standout in an emergency. The form factor of the activity monitoring device can be adapted such that the activity monitoring device can be worn and can be protruding from the skin. As such, the activity monitoring device can be given a form that avoids shapes that can be likely to snag on clothing. The activity monitoring device can have a low profile. For example, the sides of the activity monitoring device can be arranged so that they do not have a significant overhang. The sides of the activity monitoring device can be profiled, for example, as a dome, or truncated cone. The activity monitoring device can be designed to allow a low profile design, rather than a stacked design.

Chemical agents can be included in the housing material to aid with reducing friction across the housing surface. For example EPDM (ethylene propylene diene monomer rubber) can be attached to a leg or to an offloading device. The activity monitoring device can be attached to items such as boots, crutches, or a carrier. The activity monitoring device can be attached to a mobility aid. The activity monitoring device can attach to something tubular. The activity monitoring device can be attached to a carrier with a hook- and-loop fastening strap. The activity monitoring device can be attached using a zip tie or other fastener that requires no tool to secure the activity monitoring device with the fastener. The activity monitoring device can be attached by a screw. The activity monitoring device could be attached to orthopedic devices, such as external fixators, for example an Ilizarov apparatus. The activity monitoring device can be applied to a dressing, such as a wound dressing.

In order to move from a disposable to a non-disposable activity monitoring device, the housing might not have an adhesive film underneath. Due to the shedding of the skin's epithelial cells non-circumferential adhesive dressings typically have a limited wear time. Rather, in some embodiments, the activity monitoring device can use a dressing secured at its perimeter that can allow the dressing to be removed and reapplied, using the same sensor and housing. For example, the activity monitoring device can be attached using an adhesive film dressing with a hole cut out that is slightly smaller than the size of the housing. A circle or any suitable profile corresponding to a reduced version of the profile of the housing can be used. The film can tent to retain the edges of the housing, alternatively the film could be slit to have fronds, etc.

### Introduction to Wound Monitoring and Therapy

Activities of a user may be desirably monitored by an activity monitoring device for a variety of reasons including wound prevention and monitoring. In one example, the activities of a user can be monitored when the user may be prone to or already have a pressure ulcer. Information gathered by the activity monitoring device about the activities of the user can be helpful for assisting with prevention or treatment of the pressure ulcer. In addition, information gathered by the activity monitoring device about the activities can be useful for checking compliance with a treatment regimen.

Embodiments disclosed herein relate at least to apparatuses, systems, and methods for the monitoring of body loading and body position for treatment of pressure ulcers. Loading can refer to transferring or placing at least a threshold amount of force on a body part. Placing such threshold amount of force on the body part causes the body part to support weight. For example, loading of a foot can refer to transferring or placing at least a portion of the body weight (or body weight in combination with external weight) on the foot such that the foot is supporting at least such portion of the body weight. At least such portion of the body weight can serve as a threshold for determining that the foot has been loaded.

Some embodiments relate to wound monitoring or therapy for a human or animal body. Therefore, any reference to a wound herein can refer to a wound on a human or animal body, and any reference to a body herein can refer to a human or animal body. The disclosed technology embodiments may relate to preventing or minimizing damage to physiological tissue or living tissue, or to the treatment of damaged tissue (for example, a wound as described herein).

As used herein the expression "wound" may include an injury to living tissue may be caused by a cut, blow, or other impact, typically one in which the skin is cut or broken. A wound may be a chronic or acute injury. Acute wounds occur as a result of surgery or trauma. They move through the stages of healing within a predicted timeframe. Chronic wounds typically begin as acute wounds. The acute wound can become a chronic wound when it does not follow the healing stages resulting in a lengthened recovery. It is believed that the transition from acute to chronic wound can be due to a patient being immuno-compromised.

Chronic wounds may include for example: venous ulcers (such as those that occur in the legs), which account for the majority of chronic wounds and mostly affect the elderly, diabetic ulcers (for example, foot or ankle ulcers), peripheral arterial disease, pressure ulcers, or epidermolysis bullosa (EB).

Examples of other wounds include, but are not limited to, abdominal wounds or other large or incisional wounds, either as a result of surgery, trauma, sterniotomies, fasciotomies, or other conditions, dehisced wounds, acute wounds, chronic wounds, subacute and dehisced wounds, traumatic wounds, flaps and skin grafts, lacerations, abrasions, contusions, bums, diabetic ulcers, pressure ulcers, stoma, surgical wounds, trauma and venous ulcers or the like.

Wounds may also include a deep tissue injury. Deep tissue injury is a term proposed by the National Pressure Ulcer Advisory Panel (NPUAP) to describe a unique form of pressure ulcers. These ulcers have been described by clinicians for many years with terms such as purple pressure ulcers, ulcers that are likely to deteriorate and bruises on bony prominences.

Wound may also include tissue at risk of becoming a wound as discussed herein. For example, tissue at risk may include tissue over a bony protuberance (at risk of deep tissue injury/insult) or pre-surgical tissue (for example, knee tissue) that may has the potential to be cut (for example, for joint replacement/surgical alteration/reconstruction).

Some embodiments relate to methods of monitoring or treating a wound with the technology disclosed herein in conjunction with one or more of the following: advanced footwear, turning a patient, offloading (such as, offloading diabetic foot ulcers), treatment of infection, systemix, antimicrobial, antibiotics, surgery, removal oftissue, affecting blood flow, physiotherapy, exercise, bathing, nutrition, hydration, nerve stimulation, ultrasound, electrostimulation, oxygen therapy, microwave therapy, active agents ozone, antibiotics, antimicrobials, or the like.

A wound may be treated using topical pressure (such as, with negative or positive pressure therapy) or traditional advanced wound care, which is not aided by the use of applied pressure (may also be referred to as non-pressure therapy).

The use of an orthopedic device, such as a contact cast or walking boot, that offloads weight from a body part can be important for healing or preventing injury, such as a pressure ulcer, to the body part. The orthopedic device may be provided by caregiver to a patient with particular instructions to use the orthopedic device in a manner to heal or prevent injury.

### User Activity Monitoring System

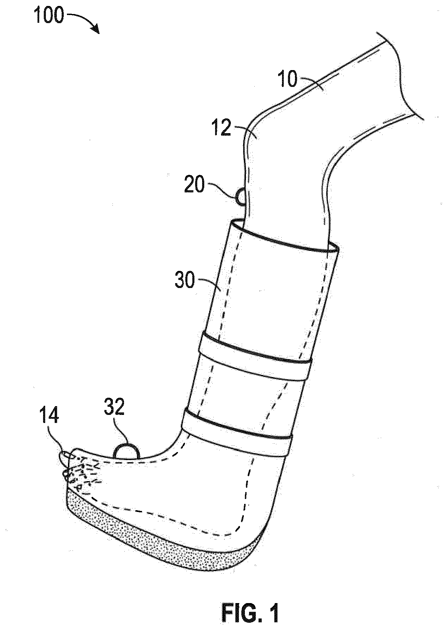

**Figure 1** illustrates a user activity monitoring system 100 including an activity monitoring device 20 attached to a body part 10. The activity monitoring device 20 can be attached to the body part 10 using a strap, adhesive, or other coupling mechanism and may be worn on or supported by the body.

The body part 10 can be a leg of a user that includes a knee 12 and a foot 14. As illustrated, in some embodiments, the activity monitoring device 20 can be supported by the body part 10 at a position between the knee 12 and the foot 14, such as proximate to the foot 14. In other embodiments, the activity monitoring device 20 can be supported by another part of the body part 12. The activity monitoring device 20 can monitor and record activities (for instance, walking, jumping, sitting, laying down, running, squatting, or standing) of the body part 10, such as from a position, movement, or orientation of the activity monitoring device 20 or one or more other sensors of the activity monitoring device 20. The activity monitoring device 20 can, for example, be used for loading monitoring of loading of the foot 14. In certain implementations, multiple body parts can be monitored by the activity monitoring device 20, and different sensors can be used for monitoring different body parts.

The body part 10 is shown wearing and partly covered by an orthopedic device 30. The orthopedic device 30 can support the body part 10 and reduce a pressure on the foot 14 when the user may be standing or engaging in other activities. A compliance monitoring device 32 can be attached to the orthopedic device 30. The compliance monitoring device 32 can be the same as or different from the activity monitoring device 20 and supported by the orthopedic device 30 using a strap, adhesive, or other coupling mechanism. The compliance monitoring device 32 can be attached to an inner surface of the orthopedic device 30 such that the compliance monitoring device 32 is disposed between the orthopedic device 30 and the skin of the patient. The compliance monitoring device 32 can be attached to an outer surface of the orthopedic device 30 such that a portion of the orthopedic device 30 is disposed between the compliance monitoring device 32 and the skin of the patient. Although not shown in Figure 1, the compliance monitoring device 32 can be attached to an orthopedic device 30 that is not worn by the patient (for example, a cane, a walker).

Although not illustrated in Figure 1, the user activity monitoring system 100 can additionally or alternatively include one or more of the activity monitoring device 20 or the compliance monitoring device 32 at other positions, such as at a position supported by the orthopedic device 30 or another part of the body part 10. These one or more additional or alternative of the activity monitoring device 20 or the compliance monitoring device 32 can be the same as or similar to the activity monitoring device 20 may monitor and record activities of the orthopedic device 30 or the another part of the body part 10.

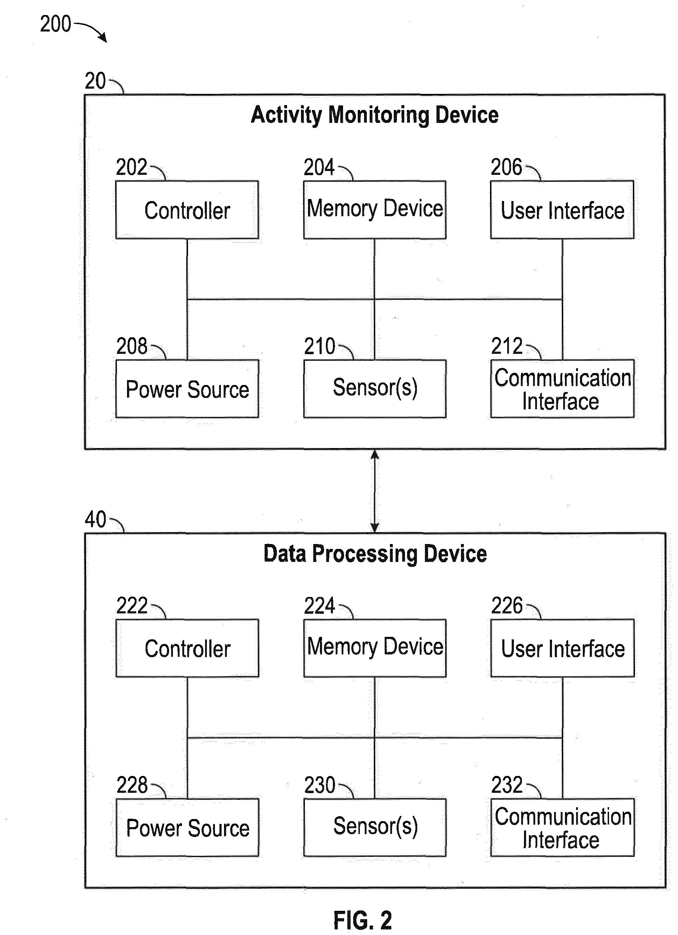

**Figure 2** illustrates components 200 of the activity monitoring device 20 and a data processing device 40. The activity monitoring device 20 can be positioned proximate to the data processing device 40 and communicate, such as wirelessly, with the data processing device 40.

The activity monitoring device 20 and the data processing device 40 can together configure a communication channel with one another to permit transfer of recorded activities or other data from the activity monitoring device 20 and the data processing device 40 or transfer of one or more commands from the data processing device 40 to the activity monitoring device 20, among other possibilities. The data processing device 40 can, for example, be a smart phone or a tablet computer.

The activity monitoring device 20 can at least partly or may be fully covered by conductive material, dielectric material, or the like (for instance, a polymer or that may approximate a conductance or capacitance of a human finger). The data processing device 40 can include a touch-sensitive display that detects the conductive or dielectric material on the activity monitoring device 20 to determine contact between the activity monitoring device 20 and the display of the data processing device 40.

As illustrated in Figure 2, the activity monitoring device 20 can include a controller 202, a memory device 204, a user interface 206, a power source 208, one or more sensors 210, and a communication interface 212 that are configured to electrically communicate with one another. The power source 208 can provide power to one or more components of the activity monitoring device 20. The components of the activity monitoring device 20 can be contained in or supported by a housing of the activity monitoring device 20 as discussed in more detail below. The data processing device 40 can include a controller 222, a memory device 224, a user interface 226, a power source 228, one or more sensors 230, and a communication interface 232 that are configured to electrically communicate with one another. The power source 228 can provide power to one or more components of the data processing device 40. The components of the data processing device 40 can be contained in or supported by a housing of the data processing device 40. In other embodiments, the activity monitoring device 20 and the data processing device 40 can include additional or alternative components than those illustrated in Figure 2.

The controller 202 can control operations of one or more other components of the activity monitoring device 20 according at least to instructions stored in the memory device 204. The controller 202 can, for instance, control monitoring of loading of the body part 10 with a weight of the body or positioning of the body part 10 and record data indicative of loading of the body part 10 or positioning of the body part 10 to the memory device 204.

The user interface 206 can include one or more output elements, such as indicators (for example, light emitting diodes) or speakers, that provide user outputs to a user. The one or more output elements can convey status information to the user like whether the activity monitoring device 20 is successfully functioning or has successfully configured communication with the data processing device 40. The user interface 206 can further include one or more input elements, such as buttons, switches, dials, or touch screens, for receiving user inputs for configuring the activity monitoring device 20. In some embodiments, the user interface 206 may have no more than one user input element, such as a button, for receiving user inputs to activate and deactivate the activity monitoring device 20 or performing one or more other functions.

The one or more sensors 210 can be used to detect and monitor a motion of the activity monitoring device 20. The one or more sensors 210 can be used to detect and monitor activities of the user of the activity monitoring device 20 that include, for instance, a loading or positioning of the body part 10. The one or more sensors 210 can include one or more accelerometers, gyroscopes, magnetometers, pressure sensors, impedance sensors, thermistors, or optical sensors, among other types of sensors. The one or more sensors 210 can be positioned proximate to the body part 10 or may be remote from the body part 10 yet usable to monitor characteristics of the body part 10.

The communication interface 212 can be used to communicate with the data processing device 40, such as via radio waves and according to a Bluetooth^{™} protocol like Bluetooth^{™} Low Energy or another protocol. The communication interface 212 can, for example, transmit device usage data like alarms, monitored loading or positioning, or changes to a monitoring or therapy program performed by the activity monitoring device 20 to the data processing device 40. The communication interface 212 can be used to receive data, including commands, from the data processing device 40.

Turning to the data processing device 40, the controller 222 of the data processing device 40 can control operations of one or more other components of the data processing device 40 according at least to instructions stored in the memory device 224. The controller 222 can, for instance, configure and control communication with the activity monitoring device 20, as well as process data received from the activity monitoring device 20 or send commands to the activity monitoring device 20. The data processing device 40 can executed one or more applications to assist with communicating with the activity monitoring device 20.

The user interface 226 of the data processing device 40 can include one or more elements that receive user inputs or provide user outputs to a user. The one or more elements of the user interface 226 that receive user inputs can include buttons, switches, dials, touch screens, or the like, and the one or more elements that provide user outputs can include indicators, screens, speakers, or the like.

The one or more sensors 230 of the data processing device 40 can be used to monitor an environment around the data processing device 40. The one or more sensors 230 can include one or more accelerometers, gyroscopes, magnetometers, pressure sensors, impedance sensors, thermistors, or optical sensors, among other types of sensors.

The communication interface 232 of the data processing device 40 can be used to communicate with the activity monitoring device 20, such as via radio waves and according to a Bluetooth^{™} protocol like Bluetooth^{™} Low Energy or another protocol. The communication interface 232 can, for example, receive device usage data like alarms, monitored loading or positioning, or changes to a monitoring or therapy program performed by the activity monitoring device 20 or transmit data like commands.

### Activity Monitoring Device Housing

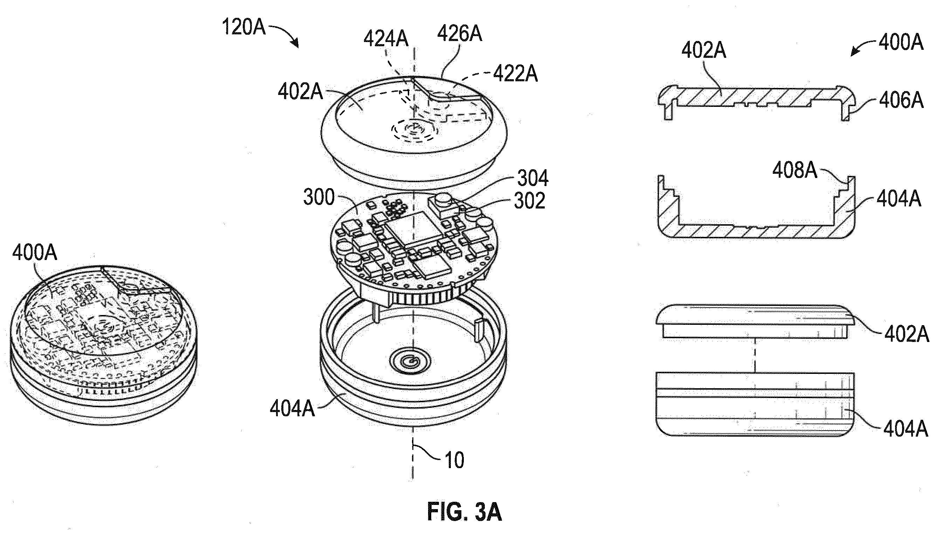

**Figure 3A** illustrates a housing 400A of an activity monitoring device 120A, which can be an example of the activity monitoring device 20. The housing 400A can be arranged to enclose a printed circuit board assembly (PCBA) 300. The housing 400A can have a cap portion 402A and a base portion 404A. The cap portion 402A and the base portion 404A can be joined together to form an enclosed space within the housing 400A. The enclosed space can be sized to at least accommodate the PCBA 300. In the embodiment shown in Figure 3A, the cap portion 402A has an inner rim 406A that nests inside an outer sidewall 408A disposed on the base portion 404A to form the enclosed space within the housing 400A. The orientation of the inner rim 406A and the outer sidewall 408A can be reversed, in some implementations, such that the inner rim 406A is disposed on the base portion 404A and the outer sidewall 408A is disposed on the cap portion 402A.

The PCBA 300 can include one or more of the components of the activity monitoring device 120A such as the controller 202, the memory device 204, the user interface 206, the power source 208, the sensor(s) 210, and the communication interface 212. In some embodiments, one or more of the components of the activity monitoring device 120A can be mounted to a portion of the activity monitoring device 120A other than the PCBA 300, such as the housing 400A. As shown in Figure 3A, the PCBA 300 can include a microswitch 302. The PCBA 300 can include more than one microswitch 302. The microswitch 302 can include a push button 304. The microswitch 302 can be activated by applying a compressive force to the push button 304.

As discussed in more detail herein, the housing 400A can be arranged such that a targeted or intended compression of the housing 400A within a particular vicinity of the microswitch 302 activates the microswitch 302. The housing 400A can include features that cause the push button 304 to be pressed when a targeted compression is applied to the housing 400A in the vicinity of the microswitch 302. The housing 400A can be arranged such that an untargeted or unintentional compression of the housing 400A outside a particular vicinity of the microswitch 302 does not activate the microswitch 302. The housing 400A can be arranged such that the housing 400A shields the PCBA 300 from compressive forces that are applied to the housing 400A.

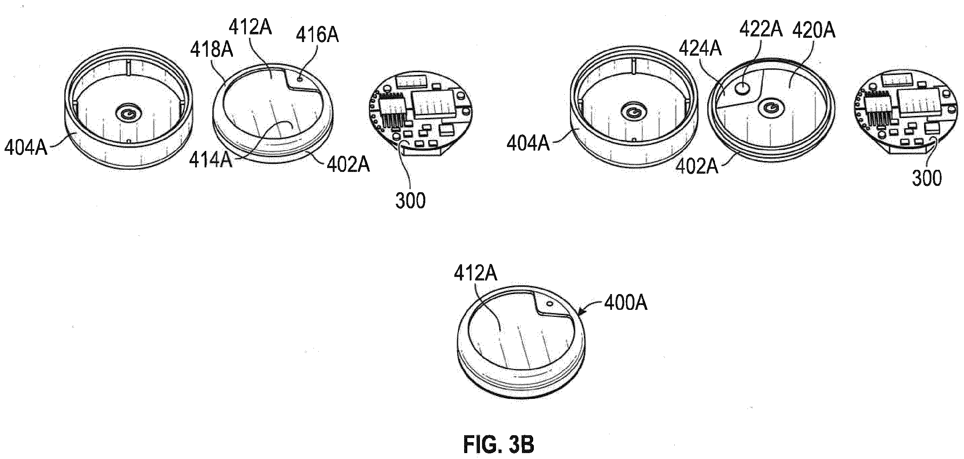

**Figure 3B** illustrates a disassembled view ofthe activity monitoring device 120A shown in Figure 3A. As shown in Figure 3B, the cap portion 402A can have an outer surface 412A that faces away from the base portion 404A when the housing 400A is assembled. The outer surface 412A of the cap portion 402A can have a recessed portion 414A and a raised portion 416A. In the illustrated embodiment, the raised portion 416A is disposed adjacent at the periphery of the cap portion 402A. The recessed portion 414A of the depicted embodiment is substantially planar and extends from the raised portion 416A to a raised rim 418A disposed at the periphery of the cap portion 402A. The depicted raised rim 418A and raised portion 416A extend away from the recessed portion 414A by substantially the same amount such that the top surfaces of the raised portion 416A and the raised rim 418A are substantially co-planar with one another where they meet. As discussed herein, the arrangement of the recessed and raised portions of the housing 400A can be differently arranged than shown in Figure 3B.

With continued reference to Figure 3B, the cap portion 402A can have an inner surface 420A that faces toward the base portion 404A when the housing 400A is assembled. The inner surface 420A of the cap portion 402A can include one or more recessed or raised portions 414A, 416A as described previously. In the illustrated embodiment, the inner surface 420A has a raised boss 422A that extends from a recessed well 424A that circumferentially surrounds the raised boss 422A.

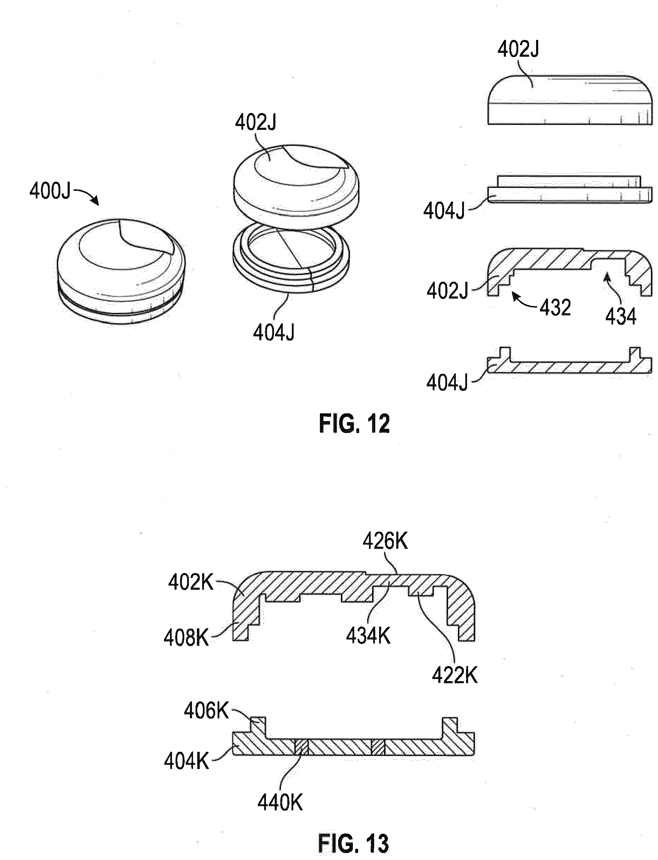

Referring back to the rendering of the cap portion 402A shown in the assembly view of Figure 3A, the cap portion 402A can include a landing pad 426A (sometimes referred to as a raised pad) that longitudinally aligns with the raised boss 422A disposed on the inner surface 420A of the cap portion 402A. The landing pad 426A can be adapted to guide the thumb of a user to the appropriate location for activating the microswitch 302A. In the embodiment shown in Figure 3A, the landing pad 426 is raised relative to the immediately adjacent portions of the outer surface 412A of the cap portion 402A. In some embodiments, the landing pad 426A can be recessed relative to the surrounding portions of the outer surface 412 of the cap 402 (see, for example, Figure 13). The housing 400A can be arranged such that the raised boss 422A is longitudinally aligned over top of the push button 304 of the microswitch 302 when the activity monitoring device 120A is assembled. The recessed well 424A can reduce the support for the landing pad 426 such that a compressive force applied to the landing pad 426A causes the landing pad 426 to move toward the base portion 404A of the housing 400A. In the illustrated embodiment, the landing pad 426A is positioned near the periphery of the cap portion 402A. In some embodiments, the landing pad 426A can be centrally located on the cap portion 402A.

The housing 400A can be arranged so that a compressive force applied to the landing pad 426A activates the microswitch 302 when the compressive force is applied substantially parallel to a longitudinal axis 10 of the activity monitoring device 120A and not when compressive force is applied substantially non-parallel to the longitudinal axis 10. For example, the raised rim 418A, the inner rim 406A, and the outer sidewall 408A can be arranged to form a support structure that distributes a compressive load away from the microswitch 302 when the compressive load is applied in a region of the housing 400A other than the landing pad 426A or in a direction other than substantially parallel to the longitudinal axis 10 of the housing 400A. The raised rim 418A, the inner rim 406A, and the outer sidewall 408A can form a thickened outer wall to resist global compression of the activity monitoring device 120A. In some embodiments, the raised rim 418A provides a stiff, hoop-like structure that transmits force to the base portion 404A through the junction of the inner rim 406A and the outer sidewall 408A, thereby bypassing transmission of the compressive load to the push button 304 of the microswitch 302. In this way, the housing 400A can be adapted to prevent the microswitch 302 from being activated by someone or something leaning on the activity monitoring device 120A.

The sensor(s) 210 can have a default or desired orientation at which the sensor(s) 210 operate. Operation of the sensor(s) 210 in the default or desired orientation may desirably, for instance, reduce a computational burden of processing data output by the sensor(s) 210 because the data may be detected relative to a default orientation, such as with respect to gravity. To facilitate the orientation and operation of the sensor(s) 210 in the default or desired orientation, the sensor(s) 210 may be positioned on the PCBA 300 at a particular orientation so that the sensor(s) 210 can be positioned with respect to the housing 400A in a certain orientation. The housing 400A can, in turn, be designed or indicated to be oriented on a user at a given orientation so that the sensor(s) 210 may be oriented during operation in the default or desired orientation. In one example, the orientation of a face of the landing pad 426A may be used to identify the default or desired orientation of the sensor(s) 210 when the cap portion 402A and the base portion 404A are coupled together and enclose the sensor(s) 210. The face of the landing pad 426A may, for instance, be initially positioned on a user to align with a direction of gravity so that the sensor(s) 210 are positioned in the default or desired orientation. Additionally or alternatively, a label placed on the housing 400A can be used to determine the default or desired orientation for the sensor(s) 210 when the sensor(s) 210 may be enclosed in the housing 400A.

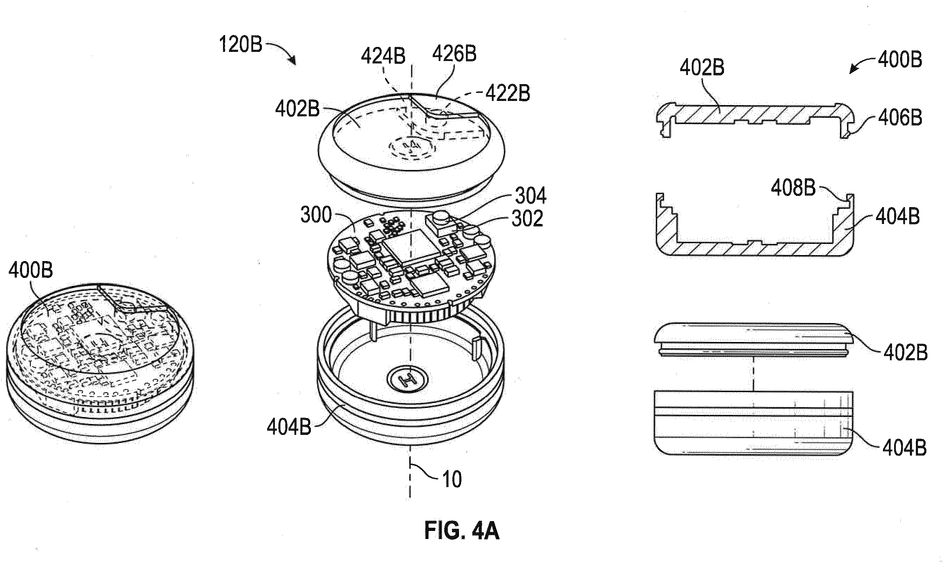

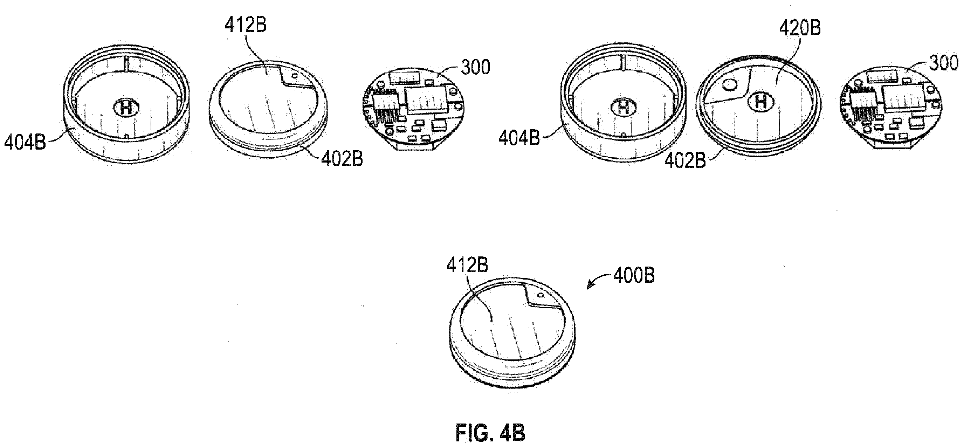

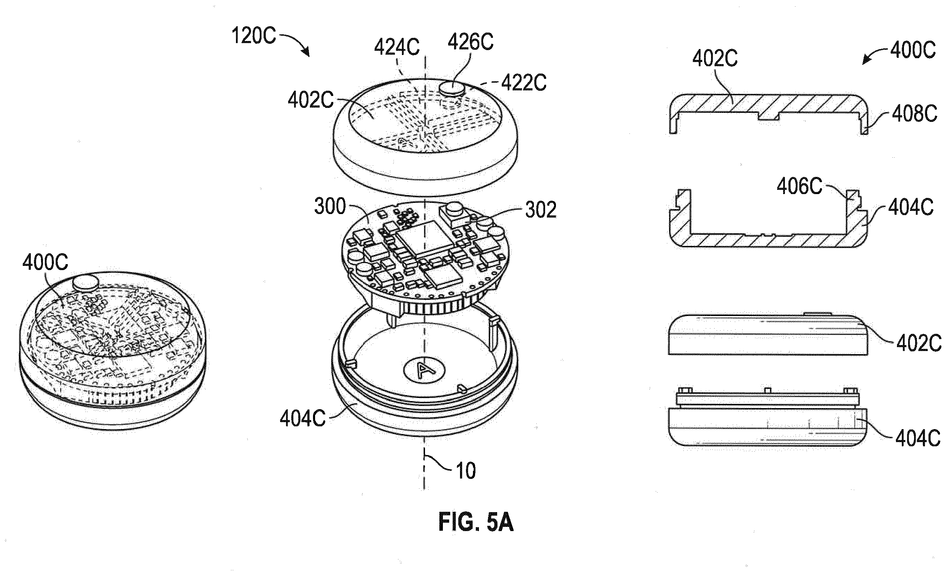

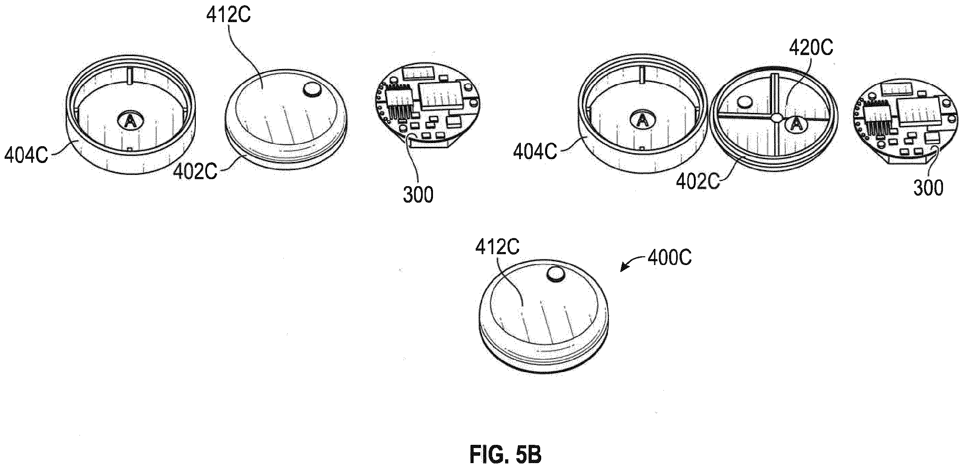

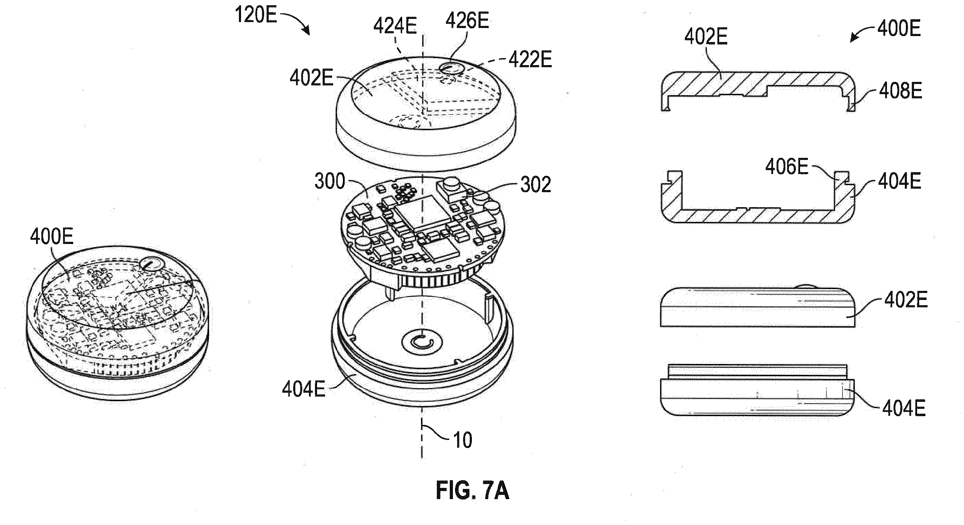

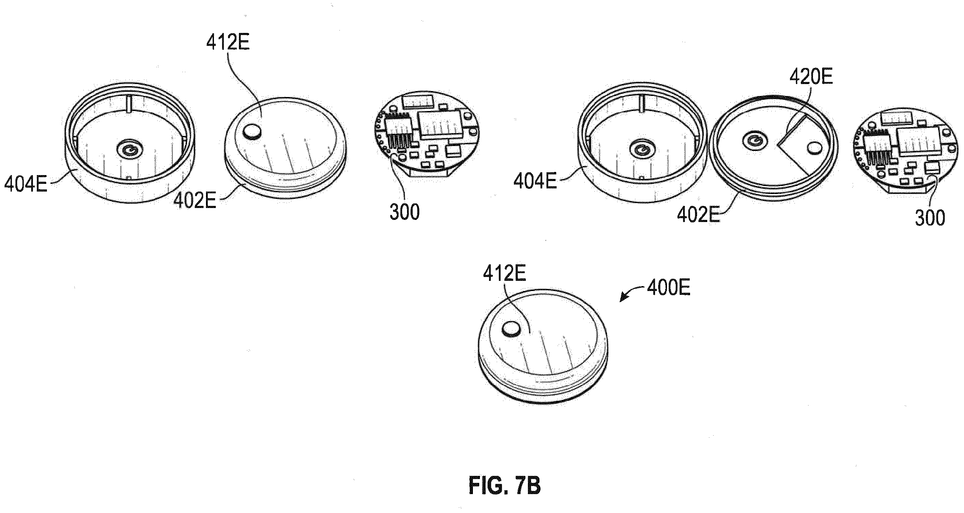

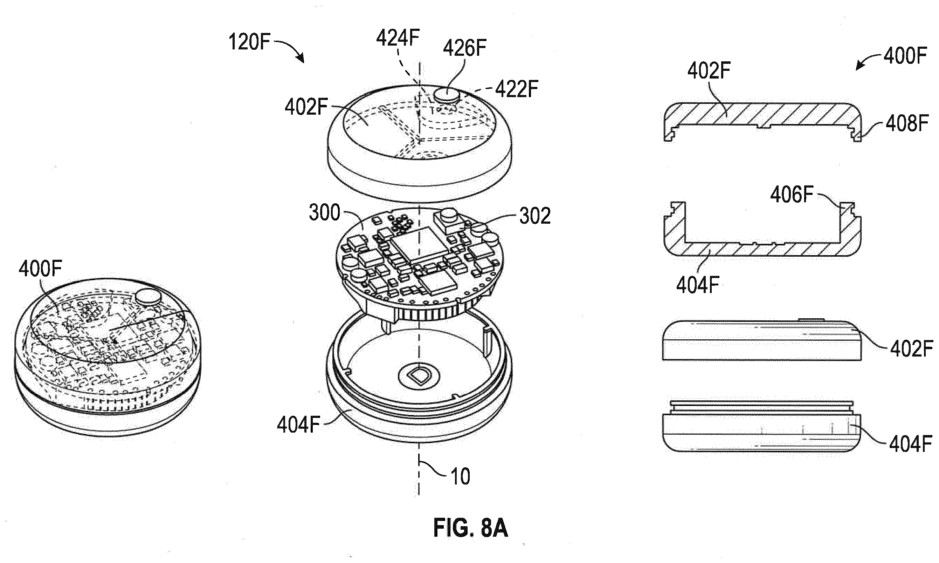

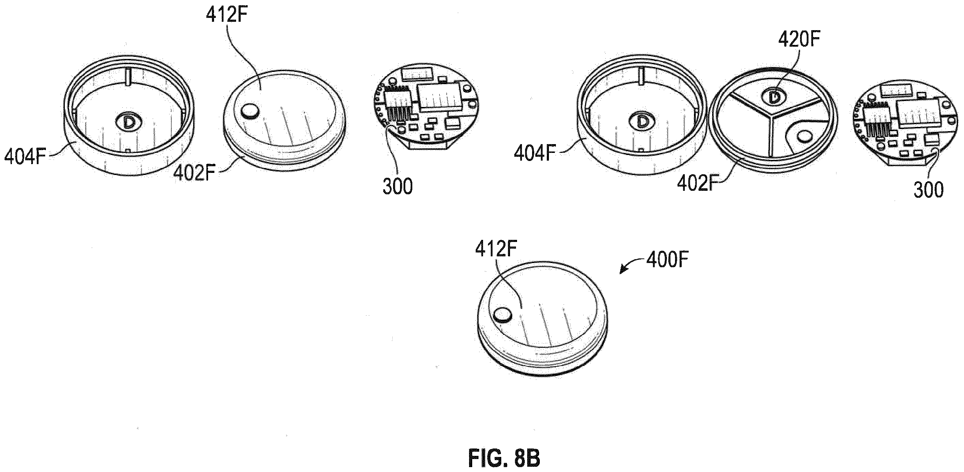

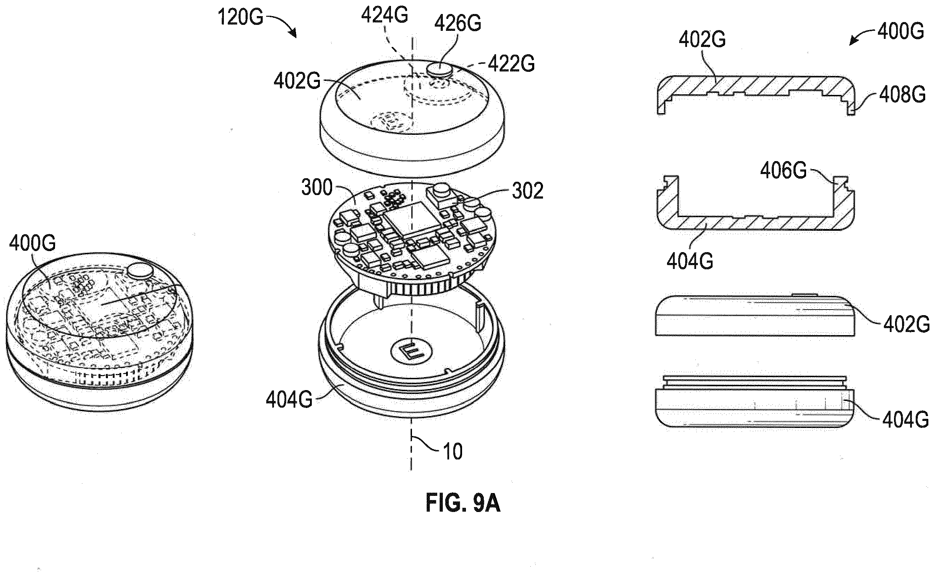

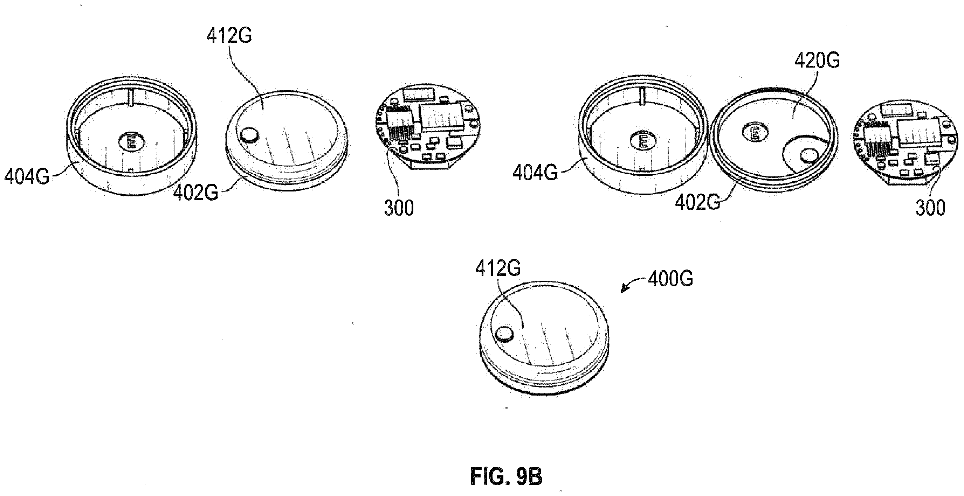

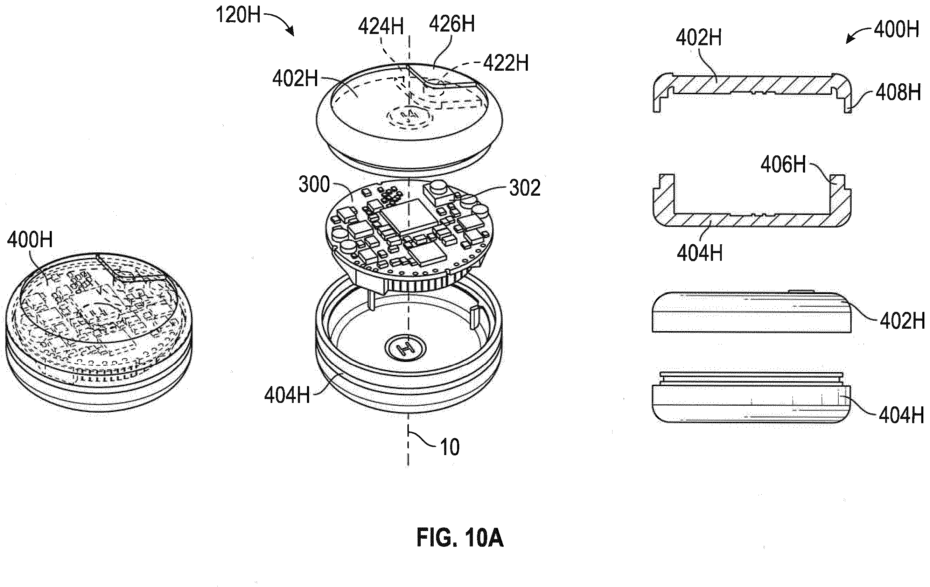

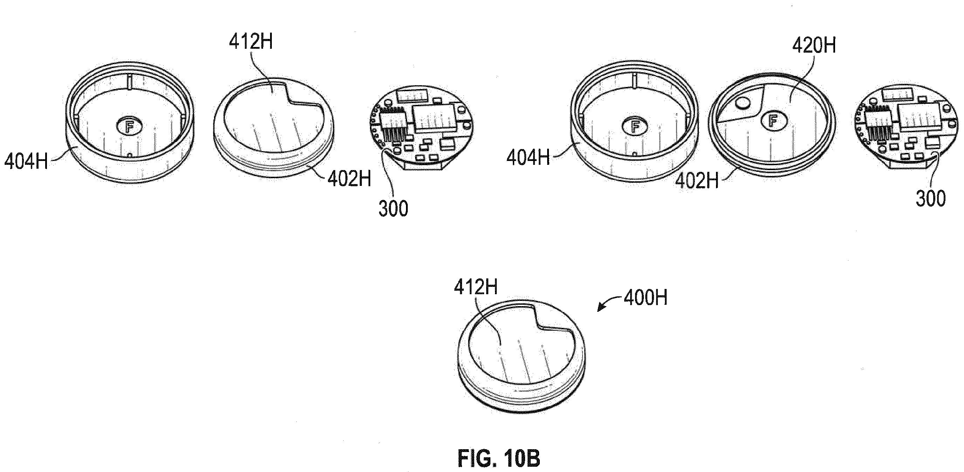

**Figures 4A to 11B** show various embodiments of the housing 400 that have different configurations of the raised and recessed portions of the housing 400. The raised and recessed portions of the housing 400 can be arranged such that the microswitch 302 is protected from being activated by an untargeted or unintended compression of the housing 400. For the sake of clarity, similar reference numbers are used within the present disclosure to denote similar features of the various embodiments. The features of the different embodiments disclosed herein can be combined with or substituted for one another to form additional embodiments within the scope of the present disclosure.

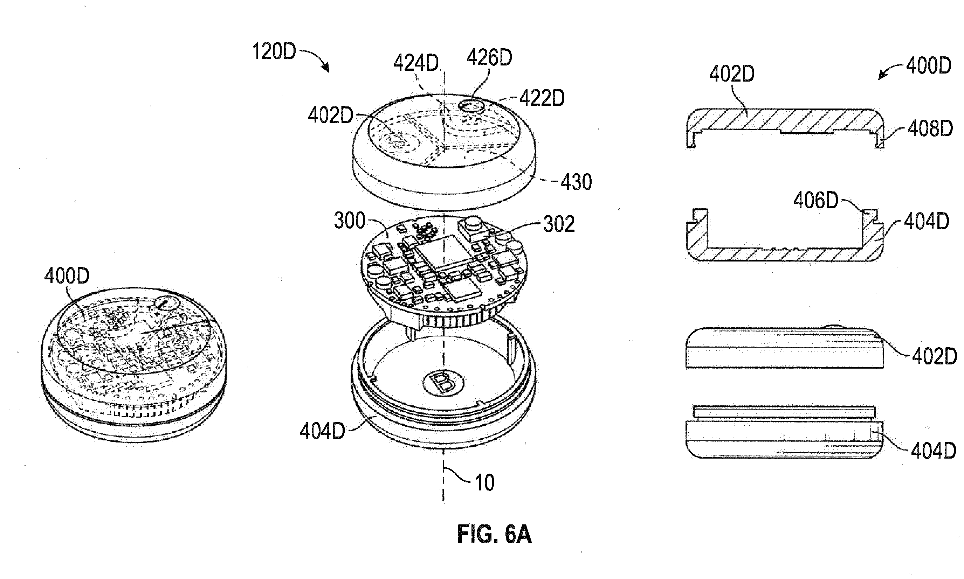

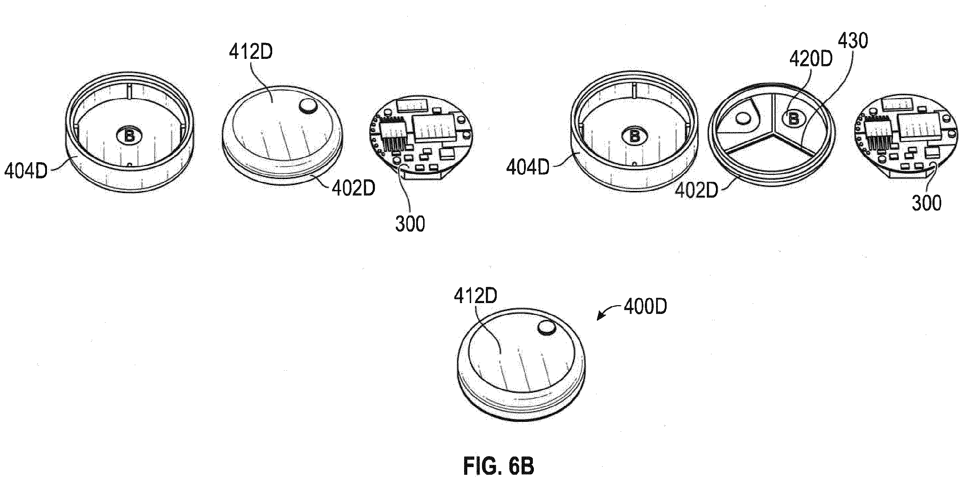

For example, as shown in Figures 6A and 6B, the housing 400D can include one or more struts 430. The struts 430 can be arranged as a thickened portion of the cap portion 402 or base portion 404 of the housing 400D. The struts 430 can be adapted to distribute or direct untargeted compressive loads away from the microswitch 302 or one or more other components within the housing 400D. The housing 400D can include one or more struts 430 disposed on the inside or outside surfaces of the cap or base portions 402, 404 of the housing 400. In the embodiment shown in Figures 6A and 6B, the housing 400D has three struts 430 that are disposed on the inside surface 420 of the cap portion 402. The three struts 430 radiate from the center point of the cap portion 402D and are substantially equally spaced apart from one another circumferentially. As shown in the embodiments disclosed herein, the housing 400D can include one or more struts 300 that are arranged differently than the equally spaced apart struts shown in Figures 6A and 6B.

**Figure 12** shows a housing 400J having a cup-shaped cap portion 402J and a dish-shaped base portion 404J. The cap portion 402J can include PCBA-seating surfaces 432, as indicated in Figure 12. The PCBA-seating surfaces 432 can help to seat the PCBA 300 into the cap portion 402. The PCBA-seating surfaces 432 can assist with positioning the PCBA 300 properly with respect the housing 400J when the activity monitoring device 120J is assembled. In some embodiments, the PCBA-seating surfaces 432 are configured such that the boss 422 aligns with the push button 304 of the microswitch 302 when the PCBA 300 is seated into the cap portion 402J of the housing 400J. In some embodiments, the PCBA-seating surfaces 432 and the cap portion 402J are arranged such that a light source (for example, LED; not shown) that is disposed on the PCBA 300 aligns with a thinned region 434J of the cap portion 402J when the PCBA 300 is seated on the PCBA-seating surfaces 432 of the cap portion 402J. The thinned region 434J can be adapted to allow the light source to be visible through the cap portion 402J when the light source is illuminated.

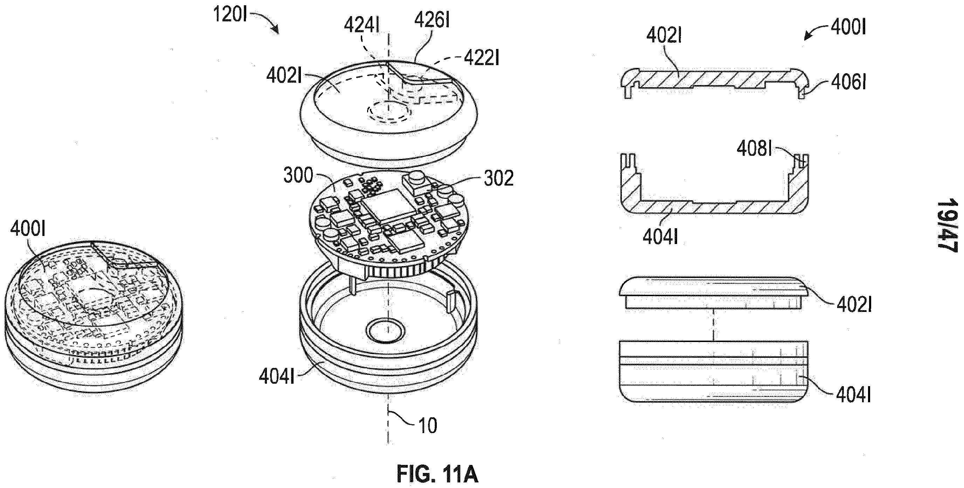

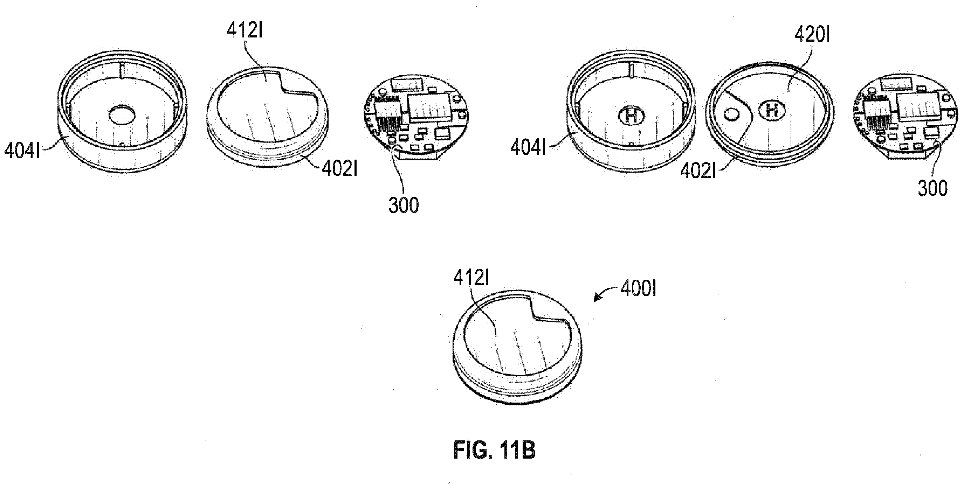

The orientation of the cup-shaped and dish-shaped portions 402J, 404J of the housing can be reversed relative to the configuration in Figure 12. For example, Figures 11A and 11B show a housing 4001 having a cup-shaped base portion 404I and a dish-shaped cap portion 402I. In some embodiments, the PCBA 300 is seated into the cupped-shaped portion of the housing 400I. In some embodiments, the PCBA 300 is seated onto the dish-shaped portion of the housing 400I. In some arrangements, proper alignment of the PCBA 300 with the housing 400J is facilitated by arranging the housing 400J such that the PCBA 300 seats onto seating surfaces 432 that are disposed on a cup-shaped cap portion 402J. This arrangement facilitates accurate assembly because the PCBA 300 may be aligned with the cap portion 402J to facilitate closure. Referring again briefly to Figure 11A, seating the PCBA 300 into a cup-shaped base portion 404I can complicate proper alignment of the PCBA 300 with the cap portion 402I because the PCBA 300 may be aligned first with the base portion 404I and then with the cap portion 402I, increasing the possibility of error compared to fitting the PCBA 300 into a cup-shaped cap portion 402, which allows a direct alignment of the PCBA 300 with the cap portion 402.

**Figure 13** shows a housing 400K having a cup-shaped cap portion 402K and a recessed landing pad 426K. The housing 400K can have an attachment feature (for example, double-sided adhesive tape) for attaching the housing 400K to an orthopedic device, a body portion of the patient, or another device or surface to be monitored. In some embodiments, the base portion 404K can include a pair of spaced-apart slots or through holes 440K. The spaced-apart slots 440K can allow a zip-tie or similar fastening device to be passed through one slot and out the other to allow the zip-tie to fasten the base portion 404K to another structure (for example, an orthopedic device). The zip-tie fasteners can be an unusual color (for example, orange) or include unique marking patterns in order to help detect whether the original zip-tie has been removed and replaced with another zip-tie. A user may remove the zip-tie to uncouple the activity monitoring device 120K from an orthopedic device in order to obscure whether the user is complying with the instructed use of the orthopedic device. In some embodiments, an adhesive may be used to secure the base portion 404K to another structure (for example, the orthopedic device). The base portion 404K can include a recess that receives an adhesive (for example, double-sided tape).

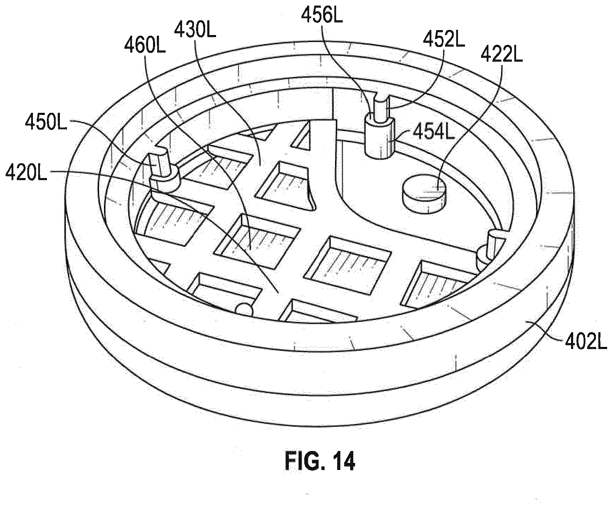

**Figure 14** shows the inner surface 420L of an embodiment of the cap portion 402L. The cap portion 402L can include one or more pins 450L. In the illustrated embodiment, the cap portion 402L includes 4 pins that are substantially equally spaced circumferentially around the periphery of the cap portion 402L. The pins 450L can be adapted to assist with the proper alignment of the cap portion 402L with the PCBA 300. The pins 450L can have a tiered-column structure in which a pin portion 452L extends from a shaft portion 454L. In some arrangements, the pin portion 452L fits into a notch on the PCBA 300 so that the PCBA 300 orients properly with respect to the cap portion 420L. The shaft portion 454L can have a seat surface 456L that rests on a cap-facing surface of the PCBA 300. The seat surface 456L can be arranged to transmit untargeted compressive forces to the portions of the PCBA 300 that are in contact with the seat surface 456L. The seat surfaces 456L can direct untargeted compressive forces away from portions of the PCBA 300 that have sensitive components that could be damaged by such compressive forces. In this way, the pins 450L can guide untargeted compressive forces to act on portions of the PCBA 300 that are in contact with the seating surfaces 456L. The inner surface 402L can include cored-out regions 460L disposed between the struts 430L. The cored-out regions 460L can be arranged to reduce the weight of the cap portion 402L. In some variants, the cored-out regions 460L and the struts 430L are arranged to provide light guides that prevent or reduce light from light sources illuminated on the PCBA 300 from diffusing into and illuminating other regions of the cap portion 402L.

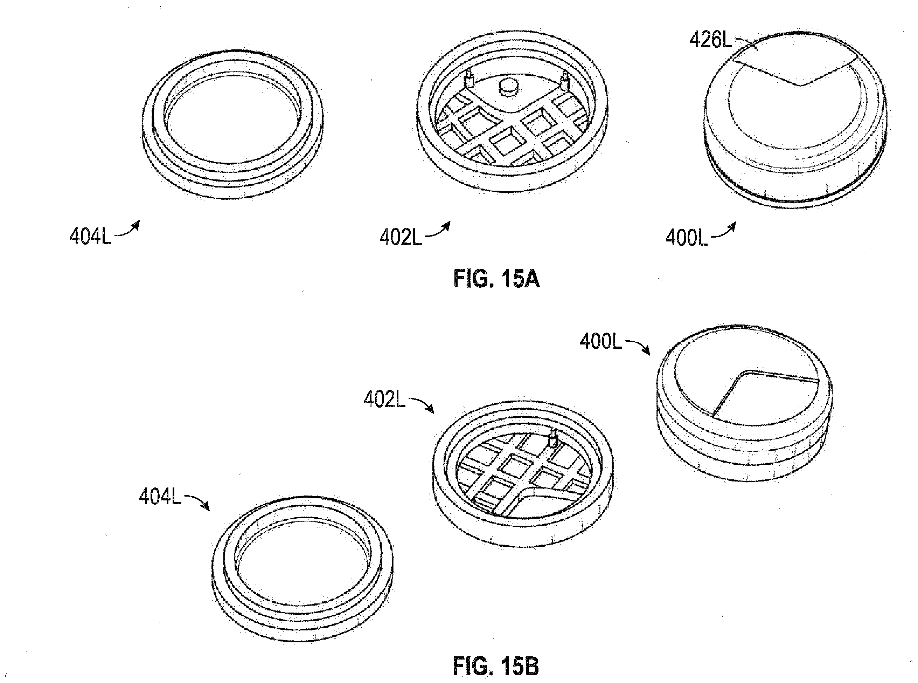

**Figures 15A and 15B** show the inner surfaces of the base portion 404L, the cap portion 402L of the housing 400L shown in Figure 14. The housing 420L can include a recessed landing pad 426L that is adapted to guide the thumb of a user to the proper location for activating a microswitch 302 of the PCBA 300 as described herein.

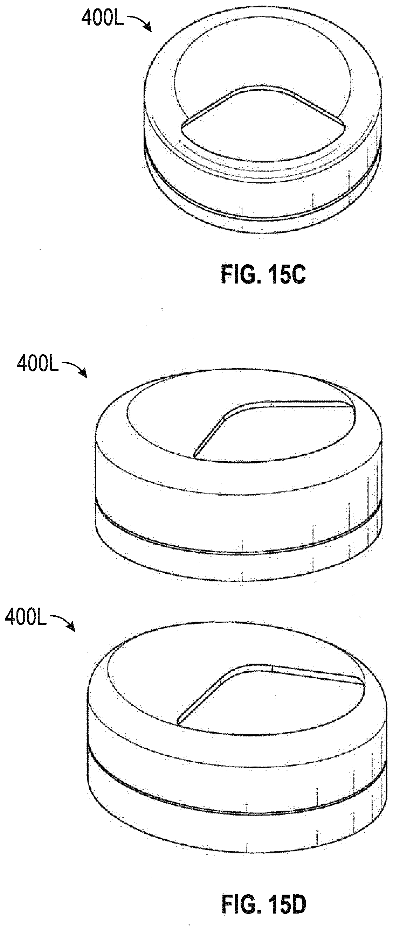

**Figures 15C and 15D** show a close up view of the housing 400L shown in Figures 15A and 15B. The top panel of Figure 15D shows the cap portion 402L assembled onto the base portion 404L without adhesive being applied to secure the cap portion 402L to the base portion 404L. As shown in the top panel of Figure 15D, a small gap between the cap portion 402L and the base portion 404L in the assembled state can accommodate the thickness for the applied adhesive that secures the cap portion 402L to the base portion 404L. In some embodiments, the gap can be adapted to enable the PCBA 300 to be clamped slightly between the cap portion 402L and the base portion 404L after the housing 400L is glued shut. The bottom panel of Figure 15D shows that the adhesive can fill the gap when the cap portion 402L and the base portion 404L are securely glued together and the housing 400L is sealed.

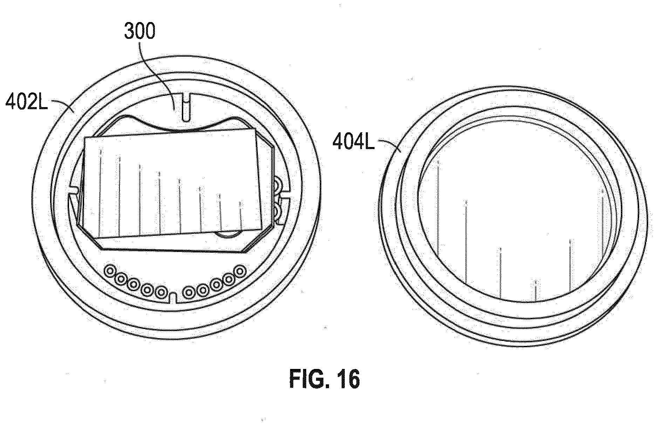

**Figure 16** shows the PCBA 300 can be seated into the cap portion 402L and can include a sensor (for example, motion sensor) or another electronic or non-electronic component (for example, wireless communication device).

### Assembly and Attachment of Activity Monitoring Device

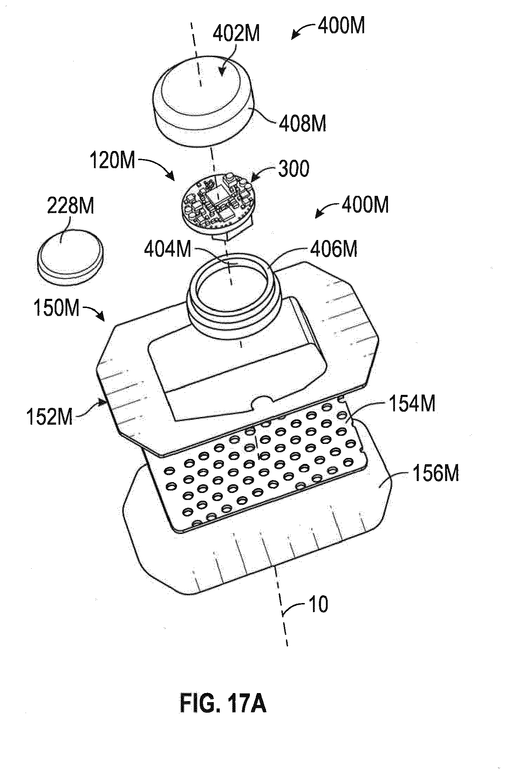

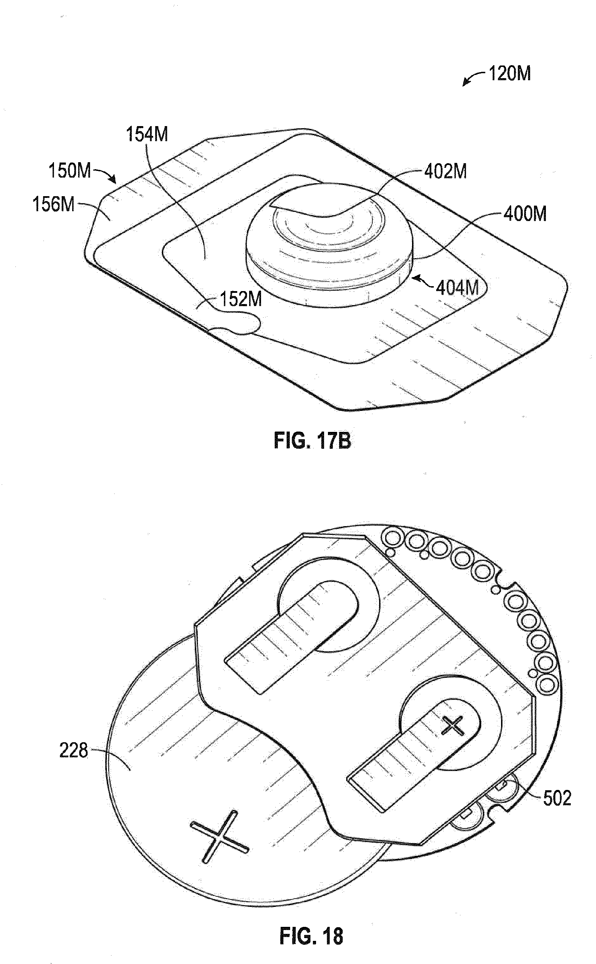

**Figures 17A** and **17B** illustrate an embodiment of an activity monitoring device 120M that includes a dressing 150M. The activity monitoring device 120M can be an example implementation of the activity monitoring device 20. As shown in Figure 17A, a housing 400M can have a cap portion 402M and a base portion 404M. The cap portion 402M and the base portion 404M can be joined together to form an enclosed space within the housing 400M. The enclosed space can be sized to at least accommodate the PCBA 300. In the embodiment shown in Figure 17A, the base portion 404M has an inner rim 406M that nests inside an outer sidewall 408M disposed on the cap portion 402M to form the enclosed space. As discussed previously, the orientation of the inner rim 406M and the outer sidewall 408M can be reversed, in some implementations, such that the inner rim 406M is disposed on the cap portion 402M and the outer sidewall 408M is disposed on the base portion 404M, such as is shown in Figure 3A.

The activity monitoring device 120M may be assembled with a power source 228M, such as a battery. The activity monitoring device 120M can be sealed to enclose the components within the housing 400M. The housing 400M can be covered with a film to mitigate against contact with the housing material.

The activity monitoring device 120M may include the dressing 150M to secure the activity monitoring device 120M to a body part or an orthopedic device. In some embodiments, the housing 400M and the dressing 150M may be pre-assembled by the manufacturer with the housing 400M adhered to the dressing 150M. In other embodiments, the housing 400M and the dressing 150M may be provided separately by the manufacturer and configured to be adhered to one another by a user. The dressing 150M can include a frame delivery layer 152M, must include a film layer 154M, and can include a release handle 156M. The release handle 156M may include or be provided with tape, such as surgical tape, which can be usable for a clinician to write information. The housing 400M may be attached to the dressing 150M in a variety of ways as discussed herein. The frame delivery layer 152M may be an upper carrier layer of the film layer 154M. The frame delivery layer 152M may be removed to expose or reveal a non-tacky upper surface of the film layer 154M. The film layer 154M may be dimensioned to cover an outer diameter of the housing 400M or a body part 110 of a patient or other object. The film layer 154M can larger than the housing 400M. The dimensions of the film layer 154 may vary. In certain embodiments, a width or length of the film layer 154 may range from 5 cm to 20 cm, such as 6 cm to 15 cm or 10 cm to 12 cm. The film layer 154M can have a width or length that is 1.25, 1.5, 1.75, 2, 2.5, or 3 times or more than a diameter or length of the housing 400M in some implementations.

**Figure 18** illustrates an embodiment of a power source 228 partially inserted into an associated mount 502. Upon full insertion of the power source 228 into the associated mount 502, the power source 228 and the associated mount 502 are ready for connecting to the PCBA 300 and insertion and sealing within the housing 400M.

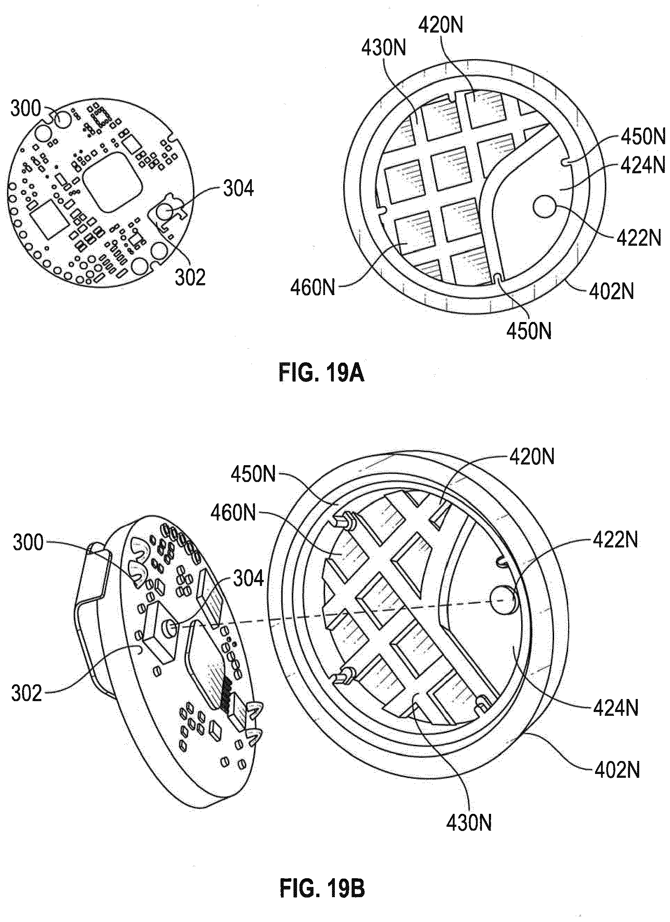

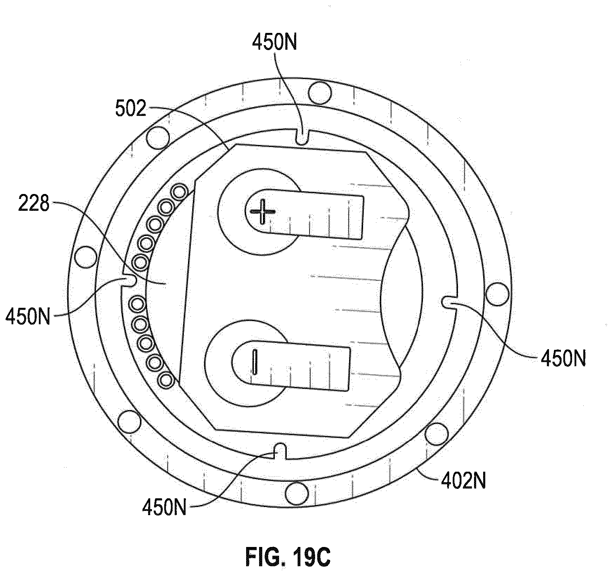

The features shown in **Figures 19A, 19B****,** and **19C** can be similar to those described with respect to Figure 14. A cap portion 402N can include one or more pins 450N. The cap portion 402N can, for example, include 4 pins that are substantially equally spaced circumferentially around the periphery of the cap portion 402N. The pins 450N can assist with the proper alignment of the cap portion 402N with the PCBA 300. The pins 450N can have a tiered-column structure in which a pin portion extends from a shaft portion. The pins 450N can have also have a straight column structure in which each of the pins 450N has a uniform diameter.

Figure 19B shows the power source 228 and the PCBA 300 being properly aligned for positioning within the cap portion 402N, and Figure 19C shows the power source 228 and PCBA 300 positioned within the cap portion 402N. As described previously, the cap portion 402N can be arranged so that the raised boss 422N is longitudinally aligned over top of the push button 304 of the microswitch 302 when the activity monitoring device 120M is assembled. The recessed well 424N can reduce the support for a raised pad on the outside surface of the cap portion 402N so that a compressive force applied to the raised pad causes the raised boss 422N to move toward the push button 304. The housing 400 can be arranged so that a compressive force applied to the raised pad activates the microswitch 302 when the compressive force is applied substantially parallel to a longitudinal axis 10 of the activity monitoring device 120M and not when compressive force is applied substantially non-parallel to the longitudinal axis 10. The cap portion 402N can have an inner surface 420N. The inner surface 420N has a raised boss 422N that extends from a recessed well 424N that circumferentially surrounds the raised boss 422N.

As shown in Figure 19C, each of the pins 450N can fit into a notch on the PCBA 300 so that the PCBA 300 orients properly with respect to the cap portion 402N. The inner surface 420N can include cored-out regions 460N disposed between struts 430N. The cored-out regions 460N can reduce the weight of the cap portion 402N. In some variants, the cored-out regions 460N and the struts 430N can provide light guides that prevent or reduce light from light sources illuminated on the PCBA 300 from diffusing into and illuminating other regions of the cap portion 402N.

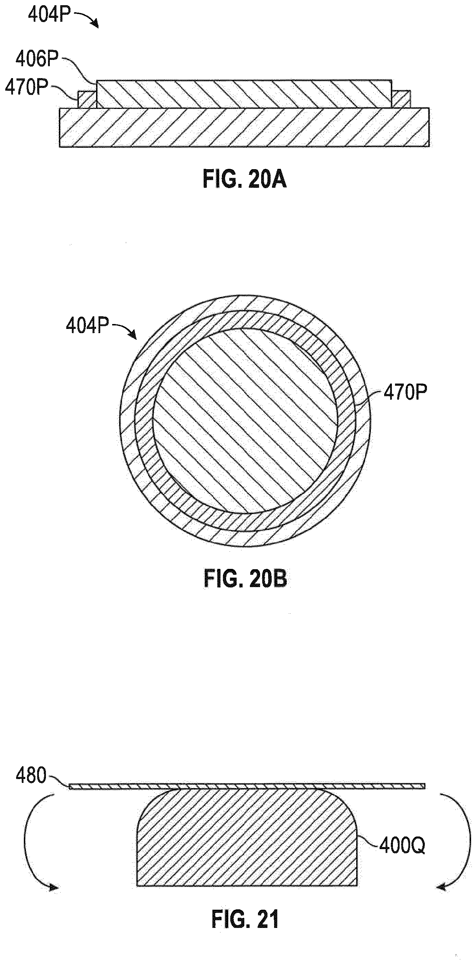

**Figures 20A** and **20B** respectively illustrate side and top views of a base portion 404P. The base portion 404P has an inner rim 406P that nests inside an outer sidewall disposed on the cap portion to form the enclosed space. An adhesive 470P is further shown as applied around a perimeter of the base portion 404P. The adhesive 470P may be applied on the horizontal surface surrounding the inner rim 406P that extends radially inwardly from the outermost wall of the base portion 404P.

Once the adhesive 470P may be applied, a cover portion can be placed on top of the base portion 404P and lightly pressed into place to enclose components. The pressure applied may be sufficient to eliminate air bubbles, as well as to minimize a distance between the base portion 404P and the cover portion that is bridged by the adhesive 470P. For instance, in some implementations, a pressure of 10N may be applied. Excess of the adhesive 470P may be wiped away using a spatula or another appropriate instrument. The adhesive 470P can, for instance, be an instant adhesive that has a low viscosity (for example, Loctite 431) and may be usable to bond plastics and elastomeric materials with relatively fast fixturing. The adhesive can air cure for one to a few minutes. A primer, such as Loctite 770 or 7239 may further be used in some implementations.

**Figure 21** illustrates a wrapping 480 positioned for application to a housing 400Q. The housing 400Q may then be covered with the wrapping 480, which may be a film, to mitigate against contact with the housing 400Q. In some embodiments, the housing 400Q may not be wrapped.

The wrapping 480 may be a circle of that is dimensioned to cover the housing 400Q. For example, the wrapping material 480 may be a circle of approximately 5 cm in diameter. The wrapping material 480 may be a film, such as a retention strip used with wound dressings, and may first be applied to the outer surface of a cap portion of the housing 400Q. The wrapping material 480 may be wrapped around the housing 400Q to cover all surfaces exposed to the environment.

The wrapping 480 may be covered by a first carrier layer and a second carrier layer, which may be made of paper. The wrapping 480, including both the first and second carrier layers, may be placed on a work surface. The first carrier layer may be removed from the wrapping 480 to expose the tacky or adhesive surface of the wrapping 480. With the exposed tacky surface facing upwards, the housing 400Q may be placed centrally on the wrapping 480. The second carrier layer may then be removed to wrap the wrapping material 480 around the housing 400Q. Figure 21 further illustrates with arrows the direction for wrapping of the wrapping 480 around the housing 400Q.

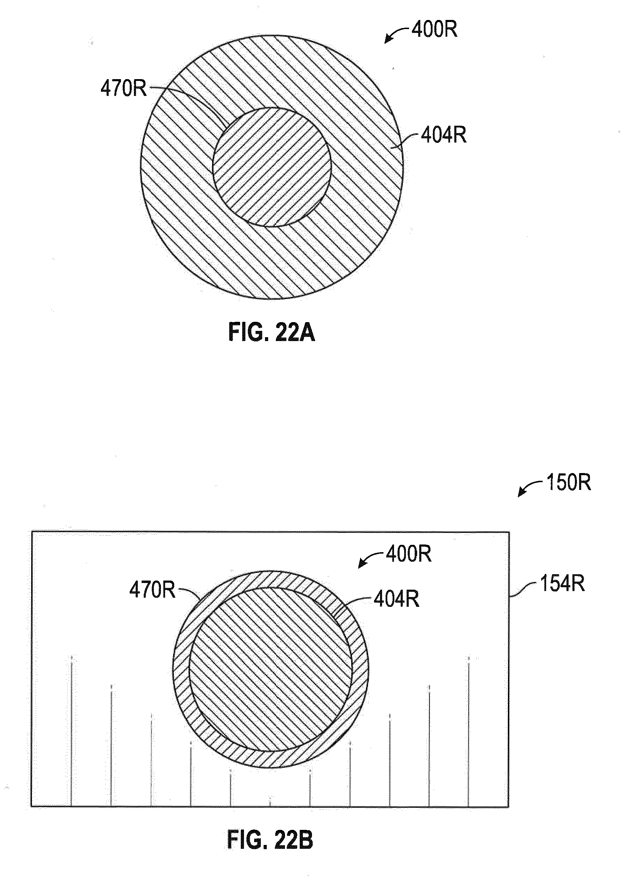

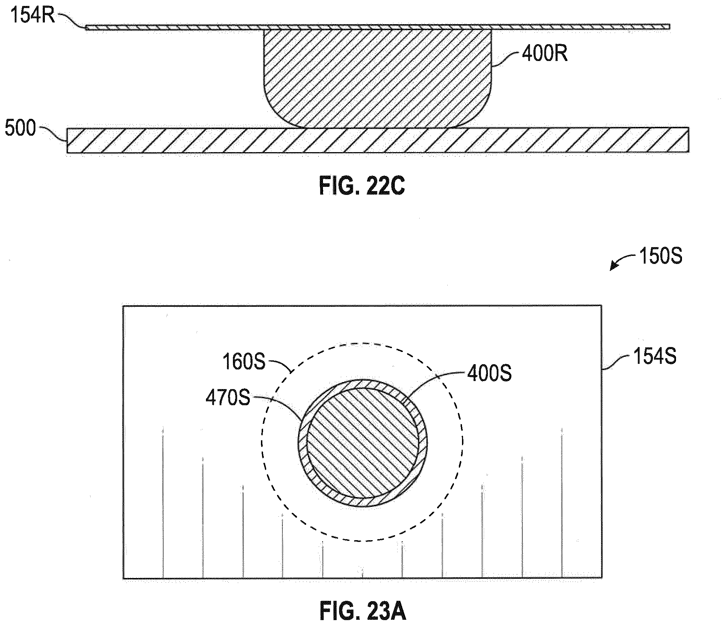

**Figures 22A, 22B****,** and **22C** illustrate a process of attaching a housing 400R to a dressing 150R. An adhesive 470R can be applied to a lower surface of a base portion 404R of the housing 400R. The adhesive 470R can be applied in a variety of ways, such as with a pinette. The adhesive 470R can be applied centrally on the base portion 404R. The adhesive 470R may, for example, be applied to approximately half of the surface area of the base portion 404R, as shown in Figure 22A.

In Figure 22B. the housing 400R can be placed on a film layer 154R of the dressing 150R.

In one example, the dressing 150R may have a 6 cm by 7 cm film layer 154R with full spread A8 adhesive on the lower surface of the film layer 154. The base portion 404R with the adhesive 470R applied may be placed on the upper surface of the film layer 154R. The base portion 404R may be placed centrally on the upper surface of the film layer 154R. Light pressure may be applied to the housing 400R against the upper surface of the film layer 154R to evenly distribute the adhesive 470R. As shown in Figure 22B, the adhesive 470R can also be applied around a perimeter of the base portion 404R to further secure the housing 400R to the upper surface of the film layer 154R. The film layer 154R may be moisture vapor permeable.

Figure 22C illustrates the housing 400R with the film layer 154R placed on a curing shelf 500, such as an ultraviolet (UV) curing shelf. The housing 400R with the film layer 154R may positioned so that the film layer 154R may be on an opposite side of the housing 400R from the curing shelf 500. The housing 400R may be placed centrally on the curing shelf 500. The housing 400R can be in physical contact with the curing shelf 500 to increase UV penetration. The housing 400R with the film layer 154R may be cured for a period of time, such as 60 seconds.

With the housing 400R adequately secured to the dressing 150R, the dressing 150R can be secured to a body part of a patient or another object, such as an orthopedic device. In some embodiments, such as described with respect to Figures 17A, a release handle 156M may be provided as a lower carrier layer on the film layer 154R. The release handle 156R may be removed to expose or reveal a tacky lower surface of the film layer 154R. The tacky lower surface of the film layer 154R may include a full spread A8 adhesive which is configured to adhere to the body part or other object.

In another example, the dressing 150R may have a 10 cm by 12 cm film layer 154R with full spread A8 adhesive on the lower surface of the film layer 154R. The housing 400R may be attached to the dressing 150R and secured to a patient or another surface using a process similar to the process described with respect to Figures 22A-22C. In other examples, the film layer 154R may have different dimensions.

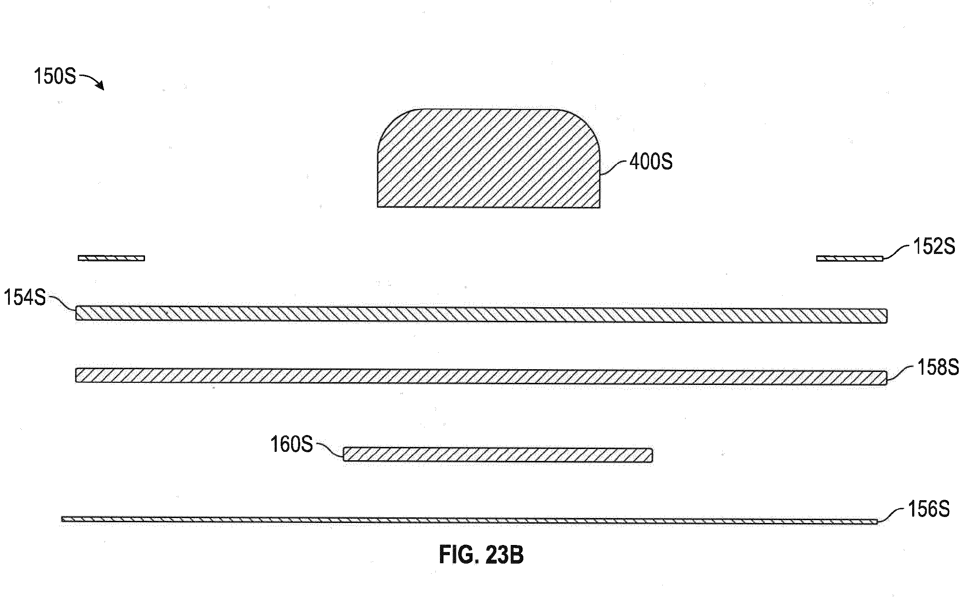

**Figures 23A** and **23B** respectively illustrate of top and side views of a housing 400S positioned on a dressing 150S. The dressing 150S can include a contact layer 160S, which may be made of silicone. The contact layer 160S may, for example, be a circular layer dimensioned (for example, with an approximately 5 cm. diameter) to underly a bottom surface of the housing 400S. The housing 400R may be attached to the dressing 150S and secured to a patient or another surface using a process similar to the process described with respect to Figures 22A-22C.

As shown in Figure 23B, the dressing 150S may include a frame delivery layer 152S, must include a film layer 154S, can include an adhesive spread layer 158S, a contact layer 160S, and a release handle 156S. The frame delivery layer 152S may be an upper carrier layer of the film layer 154S. The release handle 156S may be a lower carrier layer of the film layer 154S.

To manufacture the dressing 150S shown in Figure 23A, a previously manufactured film layer 154S with an adhesive spread layer 158S may be provided. At least a release handle 156S may already cover the adhesive spread layer 158S. The film layer 154S with release handle 156S covering adhesive spread layer 158S may be placed on a work surface with the release handle 156S facing up. The release handle 156S may be removed to expose the adhesive spread layer 158S, which can be a tacky surface. The adhesive spread layer 158S can be a full spread A8 adhesive.

A contact layer 160S, which may be made of silicone, may be provided that include an adhesive spread layer on one or both sides thereof. The contact layer 1605 may be covered with a first carrier layer or a second carrier layer on one or both sides. The first carrier layer can be removed to expose a tacky first surface of the contact layer 160S. The tacky first surface may be an adhesive spread layer covering one side of the contact layer 160S. The adhesive spread layer of the contact layer 160S may be centrally applied to the adhesive spread layer 158S. Pressure may be applied to secure the contact layer 160S to the adhesive spread layer 158S. The contact layer 160S may be perforated or moisture vapor permeable.

The second carrier layer of the contact layer 160S can be removed to expose the second surface of the contact layer 160S. The release handle 156S, which may have been previously removed as discussed above, can be reapplied to cover the contact layer 160S and the adhesive spread layer 158S of the film layer 154S. Thus, the contact layer 160S is surrounded by the adhesive spread layer 158S. The housing 400S may be attached to the dressing 150S by substantially the same or a similar process as described with respect to Figures 22A, 22B, 22C. The housing 400S may be attached to the dressing 150S before or after the assembly of the dressing as described above. The housing 400S is attached to the film layer 154S. The film layer 154S may have an adhesive spread layer 158S, which is attached to the contact layer 160S. The contact layer 160S may be sized to underlie the entirety of the housing 400S, such that the area of the contact layer 160S is at least as large as the area of a lower surface of the housing 400S. In use, the release handle 156S can be removed to expose the tacky surface of the contact layer 160S and the tacky surface of the film layer 154S, which can both be used to adhere the dressing to a body part 110 of a patient or other object. The film layer 154S may be moisture vapor permeable, and as described above the contact layer may also be moisture vapor permeable.

A re-usable or re-sealable adhesive can be used under the housing 400S and a single-use adhesive around an edge of the dressing 150S in some embodiments. The single-use adhesive may function both to provide an attachment force and serve as a tamper-evident attachment to a user. In yet other implementations, other tamper evident mechanisms such as shearing layers, stress-apparent printing, or the like may be used additionally or alternatively.

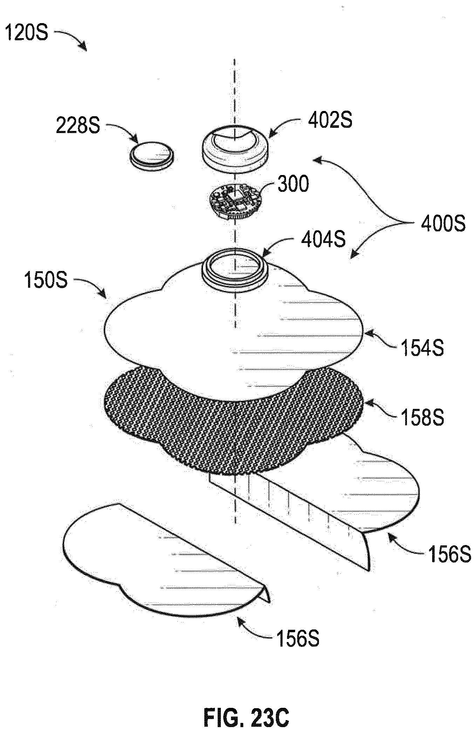

**Figure 23C** illustrates an embodiment of an activity monitoring device 120S that includes a dressing 150S. The activity monitoring device 120S can be an example implementation of the activity monitoring device 20. A contact layer (not shown in FIG. 23C) can be a silicone contact layer. The dressing 150S may be 10 cm by 10 cm. The housing 400S may be attached to the dressing 150S in a similar matter as described with respect to Figures 22A-C, 23A, and 23B.

As shown in Figure 23C, the dressing 150S may be at least partially quadrilobe shaped. The dressing 150S may have one, two, or more of the release handle 156S. The dressing 150S may include an optional frame delivery layer (not shown in FIG. 23C), must include a film layer 154S, may include an adhesive spread layer 158S, a contact layer (not shown in FIG. 23C), and two release handles 156S. The adhesive spread layer may be perforated. Alternatively, the layer 158S may be the contact layer.

The frame delivery layer may be an upper carrier layer of the film layer 154S. The release handle 156S may be a lower carrier layer of the film layer 154S, the adhesive spread layer 158S or the contact layer (not shown). The film layer 154S and the two of the release handle 156S may be cut into similar strips or shapes.

In another example, to manufacture the dressing 150S shown in Figure 23A, a previously manufactured film layer 154S with an adhesive spread layer 158S may be provided. At least a release handle 156S may already cover the adhesive spread layer 158S. The film layer 154S with release handle 156S covering adhesive spread layer 158S may be placed on a work surface with the release handle 156S facing up. The release handle 156S may be removed to expose the adhesive spread layer 158S, which can be a tacky surface. The adhesive spread layer 158S can be a full spread K5 adhesive layer. A contact layer 160S, which may be made of silicone, may be provided that include an adhesive spread layer on one or both sides thereof. The contact layer 160S may be covered with a first carrier layer or a second carrier layer on one or both sides. The first carrier layer of the contact layer 160S may be removed to expose a tacky surface of the contact layer 160S. The contact layer 160S may be placed on a work surface with the second carrier layer facing down.

The release handle 156S may be removed to expose the adhesive spread layer 158S, which may be a tacky lower surface like a K5 adhesive layer. With the film layer 154S and the contact layer 160S aligned, the adhesive spread layer 158S of may be placed onto the contact layer 160S. The second carrier layer of the contact layer 160S can be removed to expose an adhesive layer.

The release handle 156S may be perforated or folded. One of the release handles 156S can be perforated and folded and may be applied to the contact layer 160S or the adhesive layer of the contact layer 160S. The other of the release handle 156S may be applied to the contact layer 160S or the adhesive layer of the contact layer 160S. The release handle 156S may be applied so that there is an overlap between the two release handles 156S. The overlap between the two frame delivery handles may be around or at least 2.5 cm.

The housing 400S may be attached to the dressing 150S by substantially the same or a similar process as described with respect to Figures 22A-C.

The release handles 156S can be removed to expose the tacky surface of the contact layer 160S and film layer 154S, which can be adhered to a body part 110 of a patient or other object.

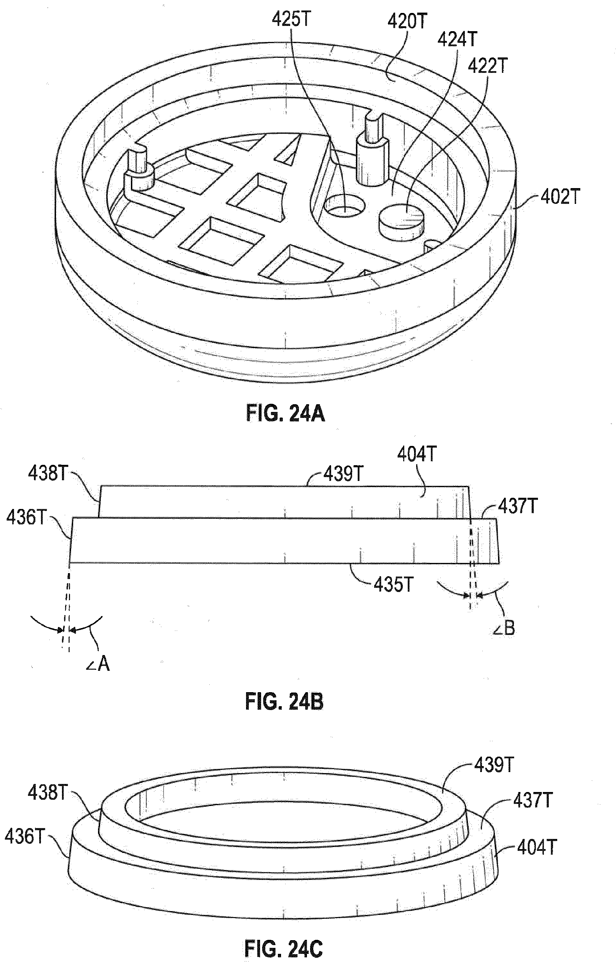

Figure 24A illustrates an embodiment of a cap portion 402T. Figure 24B and 24C illustrate side and perspective views of an embodiment of a base portion 404T. The features shown in **Figures 24A, 24B,** and **24C** can be similar to those described with respect to Figures 19A, 19B, and 19C. A cap portion 402T and a base portion 404T can generally be similar to the cap portion 402N and the base portion 404N.

In addition to the cap portion 402T having an inner surface 410T with a raised boss 422T that extends from a recessed well 424T that circumferentially surrounds the raised boss 422T, however, the recessed well 424T may include an additional recess 425T that is recessed from the recessed well 424T. The additional recess 425T can advantageously, in certain embodiments, increase a transparency of the cap portion 402T at the additional recess 425T so that the additional recess 425T passes light emitted by a light mounted the PCBA 300 through the cap portion 402T to an outside of the cap portion 402T. The additional recess 425T can moreover control a delineation or definition of light that is viewable from the outside of the cap portion 402T.

The base portion 404T can include a bottom outer surface 435T, a lower outer side surface 436T, a middle outer surface 437T, a upper outer side surface 438T, and a top outer surface 439T. The lower outer side surface 436T and the upper outer side surface 438T can respectively be angled at ∠A and ∠B relative to the top outer surface 439T. ∠A can be the same as or different from ∠B. ∠A or ∠B can, for example, be an angle within a range from 0° to 70°, such as an angle of 2°, 3°, 5°, 7°, or 10°. The corresponding parts of the cap portion 402T that are be positioned proximate to the lower outer side surface 436T and the upper outer side surface 438T when attached to the base portion 404T can be angled to match ∠A and ∠B and facilitate a fit between the cap portion 402T and the base portion 404T. The angling of the lower outer side surface 436T and the upper outer side surface 438T relative to the top outer surface 439T can desirably, in certain embodiments, increase a surface area of the bottom outer surface 435T that may be usable for attaching the base portion 404T, such as to a dressing as described herein.

The base portion 404T can be flared, flanged, or winged at the bottom outer surface 435T in some implementations. The flare, flange, or wing can facilitate use of a layer for securing the base portion 404T where the layer has a hole through which the flare, flange, or wing may not pass but through which a remainder of the base portion 404T may pass. The layer can be part of or attachable to a dressing.

The base portion 404T can have one or more surfaces with a surface roughness greater than other surfaces of the base portion 404T to facilitate and enhance the attachment of an adhesive to the surfaces with the greater surface roughness. Additionally or alternatively, the base portion 404T can have one or more textured surfaces with textures that facilitate and enhance the attachment of an adhesive to the textured surfaces.

The bottom outer surface 435T of the base portion 404T can include one or more channels (not shown) in some implementations. The one or more channels can allow air or fluid to flow and may help with presentation of a buildup of moisture under the bottom outer surface 435T. The one or more channels may reduce a lateral rigidity of the base portion 404T to improve a damping, Young's modulus, or a lateral shear modulus for the base portion 404T.

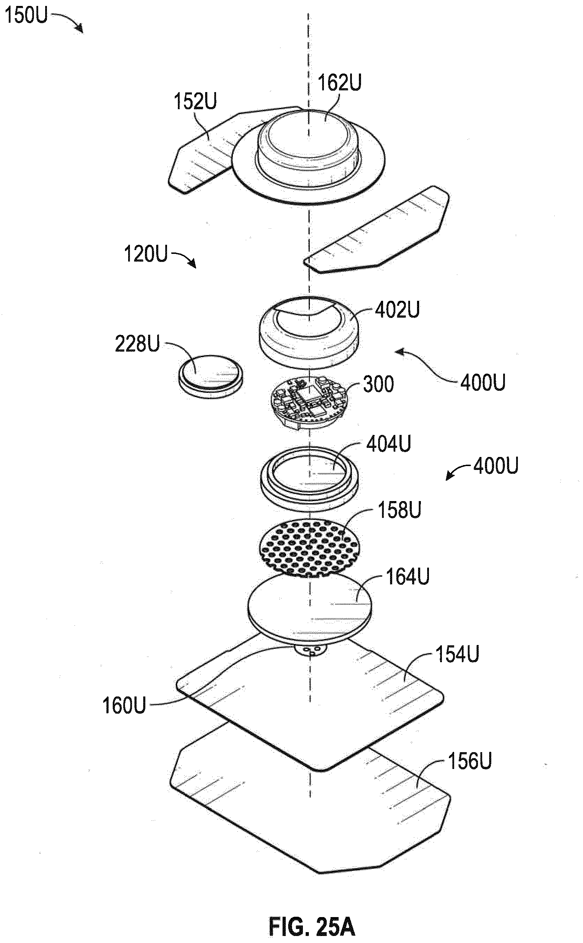

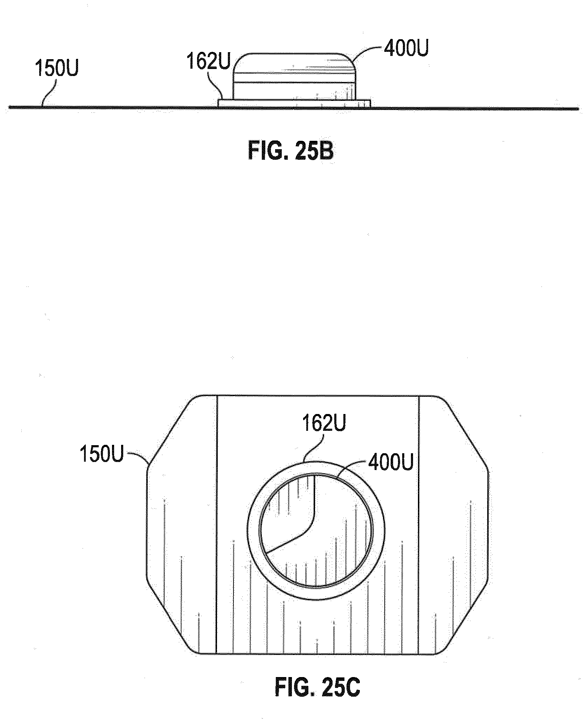

**Figures 25A****,** **25B,** and **25C** illustrate an embodiment of an activity monitoring device 120U that includes a dressing 150U. The activity monitoring device 120U can be an example implementation of the activity monitoring device 20. As shown in Figure 25A and described with respect to other embodiments herein, a housing 400U can have a cap portion 402U and a base portion 404U. The cap portion 402U and the base portion 404U can be joined together to form an enclosed space within the housing 400U. The enclosed space can be sized to at least accommodate the PCBA 300. The activity monitoring device 120U may be assembled with a power source 228U, such as a battery. The activity monitoring device 120U can be sealed to enclose the components within the housing 400U. The housing 400U can be covered with a film to mitigate against outside contact with the housing material.

The activity monitoring device 120U may include the dressing 150U to secure the activity monitoring device 120U to a body part or an orthopedic device. In some embodiments, the housing 400U and the dressing 150U may be pre-assembled by the manufacturer with the housing 400U adhered to the dressing 150U. In other embodiments, the housing 400U and the dressing 150U may be provided separately by the manufacturer and configured to be adhered to one another by a user.

The dressing 150U may include a frame delivery layer 152U, an adhesive spread layer 158U, a contact layer 160U, must include a film layer 154U, and may include a release handle 156U. The release handle 156U may include or be provided with tape, such as surgical tape, which can be usable for a clinician to write information. The frame delivery layer 152U may be an upper carrier layer of the film layer 154U.

The dressing 150U may include a spacer layer 164U for breathability by providing an air gap between the patient's skin (or another surface) and the housing 400U. The air gap may allow fluid vapour to escape. The contact layer 160U may be adhesive to aid the adherence of the spacer layer 164U to the housing 400U. The contact layer 160U maybe perforated or moisture vapor permeable, which may be advantageous for breathability.

The film layer 154U may be dimensioned to cover an outer diameter of the housing 400U or a body part 110 of a patient or other object. The film layer 154U can larger than the housing 400U. The dimensions of the film layer 154U may vary. The housing 400U may be attached to the dressing 150U in a variety of ways as discussed herein.

The activity monitoring device 120U may include a shaped film dressing 162U. The shaped film dressing 162U may be used to secure the housing 400U to the dressing 150U. The shaped film dressing 162U may be made of an adhesive film. The adhesive film may be an IV3000 dressing, such as Opsite Flexigrid dressing or another adhesive film dressing used in wound care. The adhesive can be a medical or healthcare field grade adhesive. Alternatively, the adhesive can be an adhesive film that is tolerable on human skin. The adhesive on the film can be acrylic or silicone gel. The use of the shaped film dressing 162U can enable coupling without use of an adhesive spread layer of the contact layer to adhere to adhesive spread layer of the dressing 150U. The contact layer 160U may be adhesive to aid the retention of the spacer layer 164U. The contact layer 160U may be made of silicone.

As shown in Figures 25A, 25B, and 25C, the shaped film dressing 162U may be vacuum formed over the housing 400U to hold the housing 400U to the film layer 154U of the dressing 150U. The shaped film dressing 162U may be stretched over the housing 400U. An adhesive maybe positioned between the shaped film dressing 162U and the film layer 154U. The shaped film dressing 162U and the film layer 154U may be clamped together in a brace to prevent separation. The shaped film dressing 162U may be dimensioned to receive and enclose each of the housing 400U, the spacer layer 164U, adhesive spread layer 158U, or contact layer 160U. The shaped film dressing 162U may be dimensioned to be larger than the housing 400U, the spacer layer 164U, adhesive spread layer 158U, or contact layer 160U. The shaped film dressing 162U may include a border to surround the enclosed components and secure to the film layer 154U. In the illustrated embodiment, the contact layer 160U is shown disposed between the film 154U and the spacer 164U. Additionally and alternatively, the dressing 150U can include a contact layer 160U that is disposed between the film 154U and the release handle 156U.

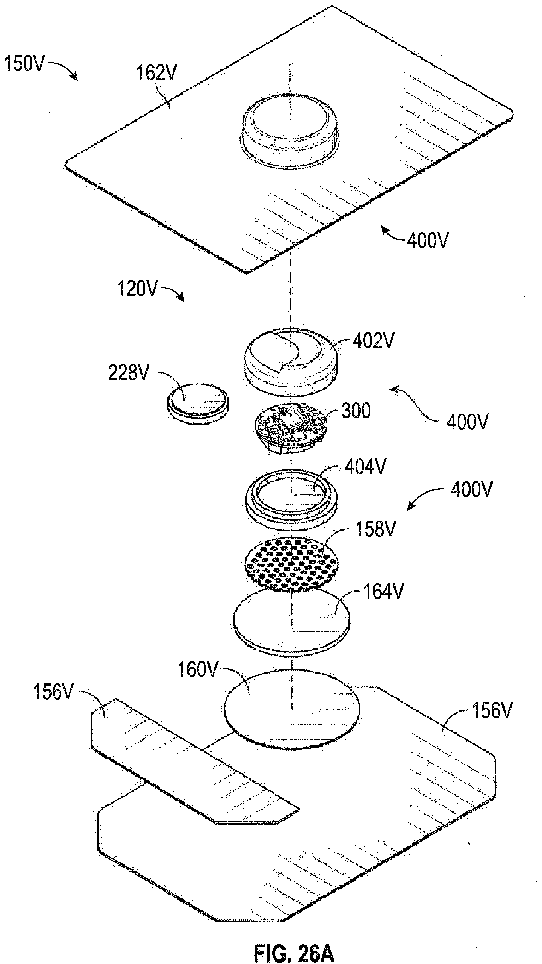

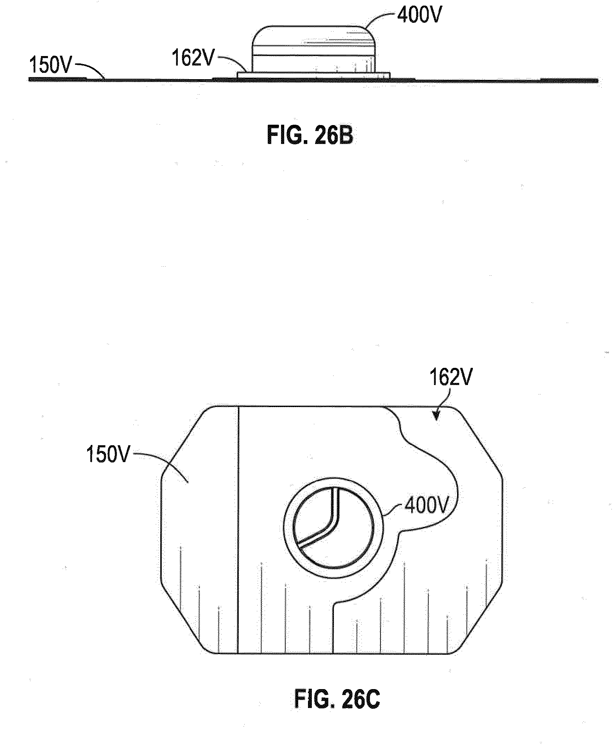

The features shown in **Figures 26A****,** **26B,** and **26C** can be similar to those described with respect to Figure 25A-25C. As shown in Figure 26A, a housing 400V can have a cap portion 402V and a base portion 404V. The cap portion 402V and the base portion 404V can be joined together to form an enclosed space within the housing 400V. The enclosed space can be sized to at least accommodate the PCBA 300. The activity monitoring device 120V may be assembled with a power source 228V, such as a battery.

The activity monitoring device 120V may include the dressing 150V to secure the activity monitoring device 120V to a body part or an orthopedic device. The dressing 150V may include an adhesive spread layer 158V, a contact layer 160V, and one or more release handles 156V. The dressing 150V may include a spacer layer 164V for breathability by providing an air gap between the patient's skin (or another surface) and the housing 400V. The air gap may allow fluid vapour to escape. The contact layer 160V may be adhesive to aid the adherence of the spacer layer 164V to the housing 400V. The contact layer 160V may be perforated or moisture vapor permeable, which may be advantageous for breathability.

The housing 400V may be attached to the dressing 150V in a variety of ways as discussed herein. The activity monitoring device 120V may include a shaped film dressing 162V. The shaped film dressing 162V may be used to secure the housing 400U or the dressing 150V to the patient's skin or another surface. The shaped film dressing 162V may also be used to secure the housing 400U to the dressing 150V. The shaped film dressing 162V may be made of an adhesive film. The adhesive film may be an IV3000 dressing, such as Opsite Flexigrid dressing or another adhesive film dressing already used in wound care. The adhesive can be a medical or healthcare field grade adhesive. The adhesive can be an adhesive film that is tolerable on human skin. The adhesive on the film can be acrylic or silicone gel. The use of the shaped film dressing 162V can enable coupling without use of an adhesive spread layer of the contact layer to adhere to adhesive spread layer of the dressing 150V. The contact layer 160V may be adhesive to aid the retention of the spacer layer 164V. The contact layer 160U may be made of silicone.

As shown in Figures 26A, 26B, 26C, the shaped film dressing 162V may be vacuum formed over the housing 400V to enclose the housing 400V and the dressing 150V. The shaped film dressing 162V may be stretched over the housing 400V. An adhesive may be positioned between the shaped film dressing 162V and the film layer 154V. The shaped film dressing 162V and the film layer 154V may be clamped together in a brace to prevent separation. The shaped film dressing 162V may be dimensioned to receive and enclose both the housing 400U and the dressing 150V as well as to adhere to the patient's skin or another surface. The shaped film dressing 162V may further be dimensioned to be larger than the dressing 150V to surround the enclosed components and secure to the housing 400V and the dressing 150V to the patient or another surface. The shaped film dressing 162V may include a border to surround the enclosed components and secure to the housing 400V and the dressing 150V to the patient's skin or another surface.

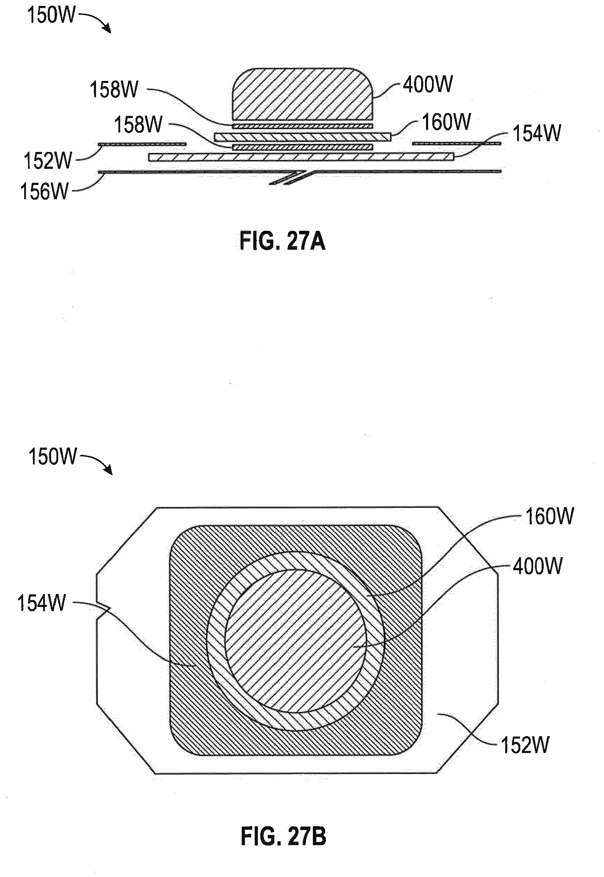

**Figures 27A** and **27B** illustrate an embodiment of a housing 400W and a dressing 150W and show an approach for coupling the housing 400W to the dressing 150W. The dressing 150W can include a frame delivery layer 152W, must include a film layer 154W, and may include a release handle 156W. The housing 400W may be secured to the dressing 150W with the use of two adhesive spread layers 158W. The dressing 150W may include a contact layer 160W positioned between the two adhesive spread layers 158W. One adhesive spread layer 158W may be positioned between the housing 400W and the contact layer 160W. One adhesive spread layer 158W may be positioned between the contact layer 160W and the film layer 154W.

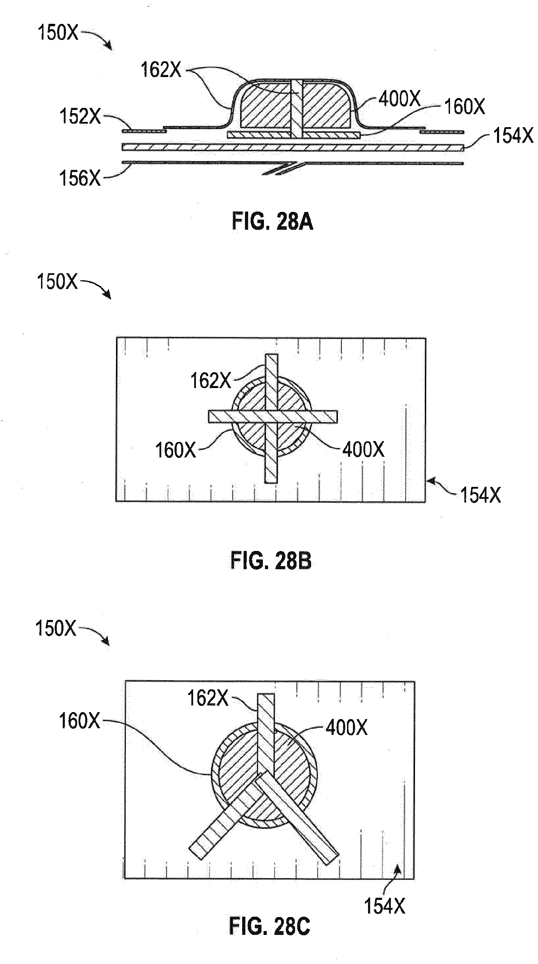

**Figures 28A, 28B,** and **28C** illustrate an embodiment of a housing 400X and a dressing 150X and show an approach for coupling the housing 400X to the dressing 150X. The dressing 150X can include a frame delivery layer 152X, must include a film layer 154X, and may include a release handle 156X. The dressing 150W may include a contact layer 160X positioned between the housing 400X and the film layer 154X. The housing 400X may be secured to the dressing 150Y with the use of one or more strips of adhesive film dressing 162X. Figures 28B and 28C illustrate different configurations of the one or more strips of adhesive film dressing 162X positioned over the housing 400X. As shown in Figure 28B, two strips of film dressing 162X may be substantially perpendicular to each other over the housing 400X. As shown in FIGURE 28C, three strips of film dressing 162X may extend radially with respect to each other over the housing 400X. The adhesive film dressing 162X may be an IV3000 dressing, such as Opsite flexigrid dressing or an adhesive film dressing used in wound care.

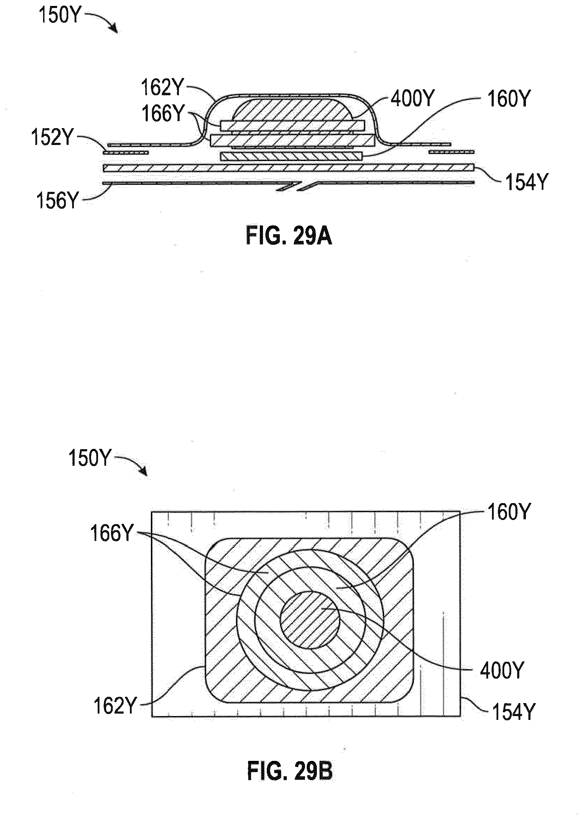

**Figures 29A** and **29B** illustrate an embodiment of a housing 400Y and a dressing 150Y and show an approach for coupling the housing 400X to the dressing 150X. The dressing 150Y can include a frame delivery layer 152Y, must include a film layer 154Y, and may include a release handle 156Y. The dressing 150Y may include a contact layer 160Y positioned between the housing 400Y and the film layer 154Y. The housing 400Y may be secured to the dressing 150Y with a foam 166Y positioned around the perimeter of the housing 400Y. The foam 166Y may be donut shaped such that the foam 166Y is round with a hole in the center to receive the housing 400Y. One or more layers of foam 166Y may be used to secure the housing 400Y to the dressing 150Y. The housing 400Y may be secured to the dressing 150Y with a film dressing 162Y. The film dressing 162Y may be adhesive, such as an IV3000 dressing, such as Opsite flexigrid dressing or another adhesive film dressing used in wound care. The film dressing 162Y may be vacuumed over the housing 400X or the dressing 150Y similar to as described with respect to other embodiments herein. The film dressing 162Y may be dimensioned to fit over the housing 400Y, the foam 166Y, and at least part of the dressing 150Y.

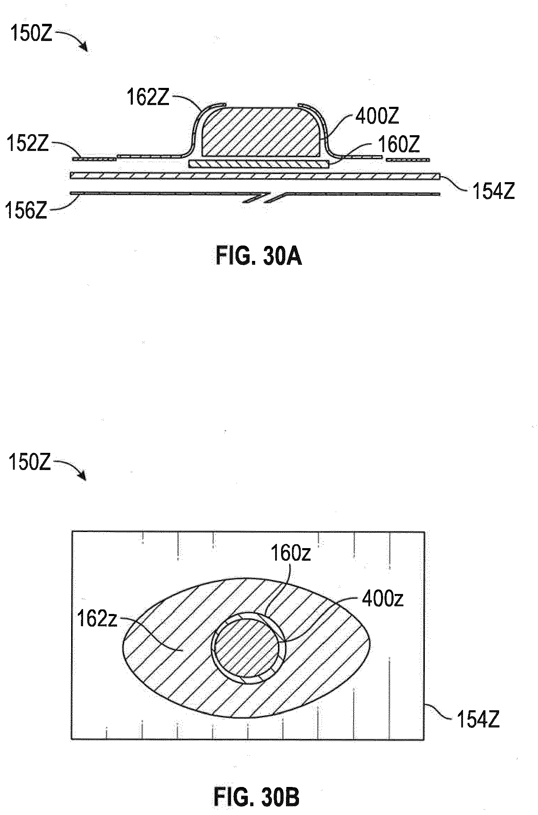

**Figures 30A** and **30B** illustrate an embodiment of a housing 400Z and a dressing 150Z and show an approach for coupling the housing 400Z to the dressing 150Z. The dressing 150Z can include a frame delivery layer 152Z, must include a film layer 154Z, and may include a release handle 156Z. The dressing 150Z may include a contact layer 160Z positioned between the housing 400Z and the film layer 154Z. The housing 400Z may be secured to the dressing 150Z with a film dressing 162Z. The film dressing 162Z may be adhesive, such as an IV3000 dressing, such as Opsite Flexigrid dressing or another adhesive film dressing used in wound care. The film dressing 162Z may be dimensioned to fit over the housing 400Y and at least part of the dressing 150Z. The film dressing 162Z may have a cut out hole to reduce strain of the film dressing 162Z over the height of the housing 400Z.

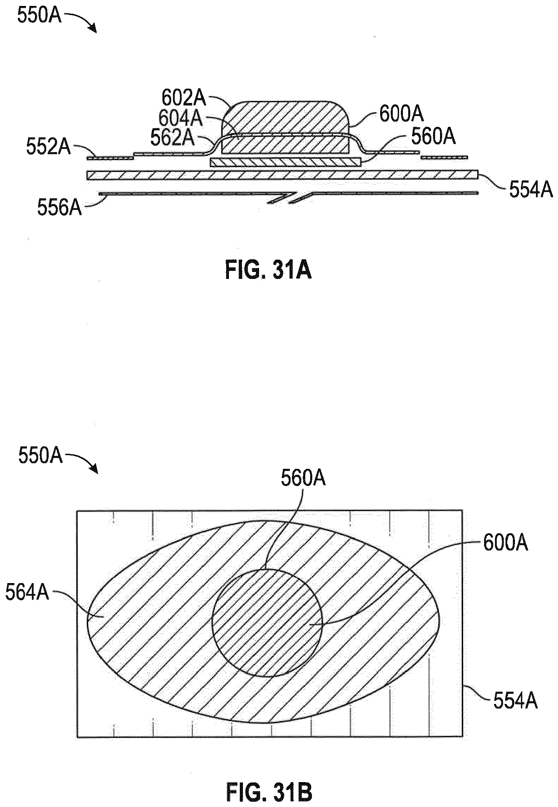

**Figures 31A** and **31B** illustrate an embodiment of a housing 600A and a dressing 550A and show an approach for coupling the housing 600A to the dressing 550A. The dressing 550A can include a frame delivery layer 552A, must include a film layer 554A, and may include a release handle 556A. The dressing 550A may include a contact layer 560A positioned between the housing 600A and the film layer 554A. The housing 600A may be secured to the dressing 550A with a film dressing 562A. The film dressing 562A may be adhesive, such as an IV3000 dressing, such as Opsite flexigrid dressing or another adhesive film dressing used in wound care. The film dressing 562A may be dimensioned to fit over the base portion 600A and at least part of the dressing 550A. The film dressing 562A may be positioned between the cap portion 602A and the base portion 604A of the housing 600A. The film dressing 562A may be initially vacuumed over the base portion 604A of the housing 600A or the dressing 550A similar to as described with respect to other embodiments herein. The film dressing 562A being positioned between the cap portion 602A and the base portion 604A of the housing 600A may reduce the strain of the film dressing 562A as it reduces the height of the film dressing 562A. The position of the film dressing 562A between the cap portion 602A and the base portion 604A may secure the film dressing 562A in place.

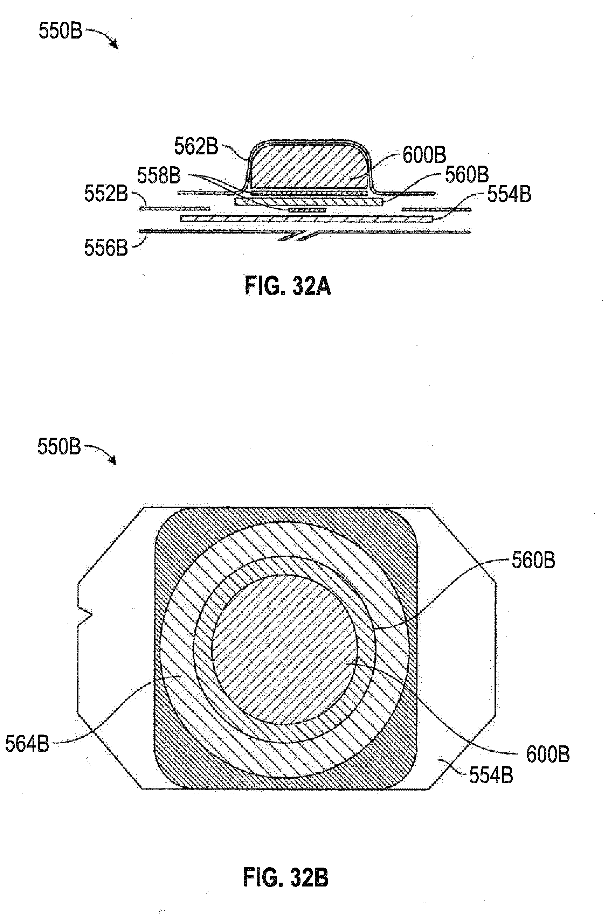

**Figures 32A** and **32B** illustrate an embodiment of a housing 600B and a dressing 550B and show an approach for coupling the housing 600B to the dressing 550B. The dressing 550B can include a frame delivery layer 552B, must include a film layer 554B, and can include a release handle 556B. The dressing 550B may include a contact layer 560B positioned between the housing 600B and the film layer 554B. The housing 600B may be secured to the dressing layer 550B with the use of two adhesive spread layers 558B. One adhesive spread layer 558B may be positioned between the housing 600B and the contact layer 560B. One adhesive spread layer 558B maybe positioned between the contact layer 560B and the film layer 554B. The housing 600B and the dressing 550B may be secured to the patient or another surface with a film dressing 562B. The film dressing 562B may secure the housing 600B to the dressing 550B. The film dressing 562B may be adhesive, such as an IV3000 dressing, such as Opsite Flexigrid dressing or any other adhesive film dressing already used in wound care. The film dressing 562B may be vacuumed over the housing 600B or the dressing 550B similar to as described with respect to other embodiments herein. The film dressing 562B may be dimensioned to fit over the housing 600B and the dressing 550B to adhere to the patient's skin or another surface.

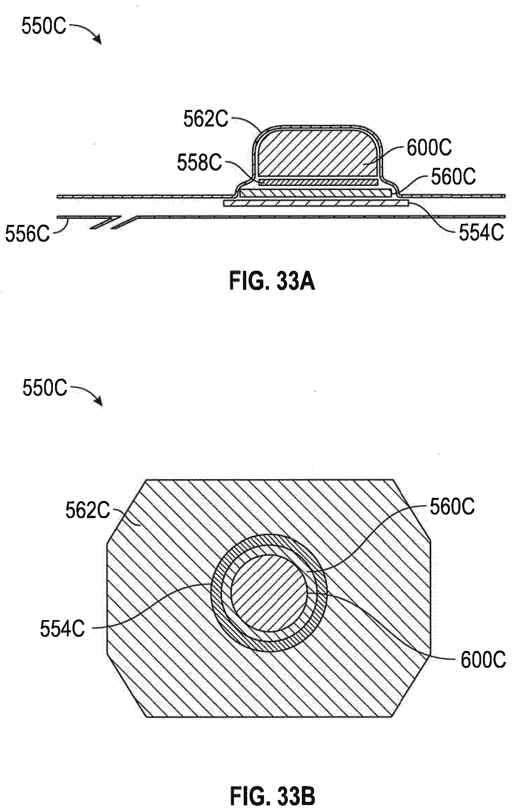

**Figures 33A and 33B** illustrate an embodiment of a housing 600C and a dressing 550C and show an approach for coupling the housing 600C to the dressing 550C. The dressing 550C can include a frame delivery layer 552C, must include a film layer 554C, and may include a release handle 556C. The dressing 550C may include a contact layer 560C positioned between the housing 600C and the film layer 554C. The housing 600C may be secured to the dressing layer 550C with the use of an adhesive spread layer 558C. The housing 600C and the dressing 550C may be secured to the patient or another surface with a film dressing 562C. The film dressing 562C may secure the housing 600C to the dressing 550C. The film dressing 562C may be adhesive, such as an IV3000 dressing, such as Opsite flexigrid dressing or any other adhesive film dressing already used in wound care. The film dressing 562C may be vacuumed over the housing 600C or the dressing 550C similar to as described with respect to other embodiments herein. The film dressing 162CC may be dimensioned to fit over the housing 600C and the dressing 550C to adhere to the patient's skin or another surface.

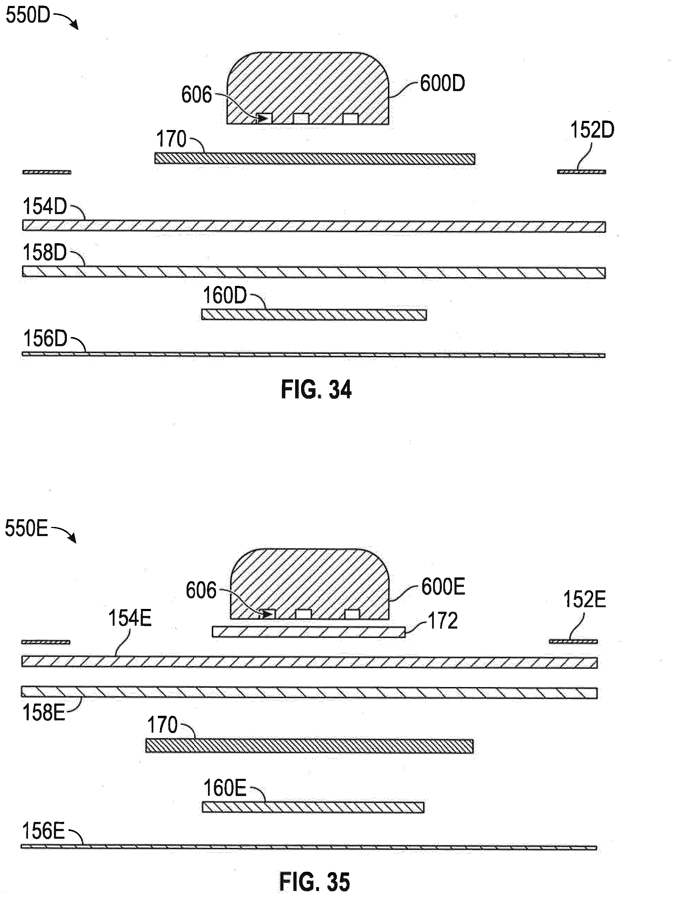

**Figure 34** illustrates an embodiment of a housing 600D and a dressing 550D and shows an approach for coupling the housing 600D to the dressing 550D. The housing 600D can include fluid handling features, as described herein. In some aspects, the fluid handling features can facilitate wicking moisture away from the interface of the housing 600D and the dressing 550D. Wicking moisture away from the housing-dressing interface can promote maintaining the attachment of the housing 600D to the dressing 550D by avoiding moisture accumulating under the housing 600D and weakening the adhesives.

With continued reference to Figure 34, the housing 600D can include one or more grooves or channels 606 on the outer surface of the housing 600D that faces the dressing 550D when the housing 600D is coupled to the dressing 550D. The channels 606D can be adapted to provide moisture handling capability through the housing. The channels 606 can provide a pathway that allows moisture to travel from underneath the housing 600D to the periphery of the housing 600D. The channels 606 can be included in any of the housings described herein. In the illustrated embodiment, the channels 606 are shown on the base portion of the housing 600D. In some variants, the channels 606 can be disposed on a ring or doughnut-shaped structure that is attached to the bottom surface of the base portion of the housing. In some variants, the entire housing has a toroidal or doughnut-like shape such that an open flow pathway is provided through the central core of the toroidal or doughnut-like shaped housing 600D.

As shown in Figure 34, the dressing 550D can include a fluid handling layer 170. The fluid handing layer 170 can be a wicking or acquisition distribution layer (ADL), a foam layer, or a spacer or transmission layer. The ADL can be configured to horizontally wick fluid such as wound exudate as it is absorbed upward through the layers of the dressing 550D. In some aspects, the ADL may comprise viscose, polyester, polypropylene, cellulose, or a combination of some or all of these, and the material may be needle-punched. In some aspects, the ADL may comprise polyethylene in the range of 40-150 grams per square meter (gsm). In some aspects, the ADL may have a thickness of 1.2 mm or about 1.2 mm, or may have a thickness in the range of 0.5 mm to 3.0 mm, or about 0.5 mm to about 3.0 mm.

The ADL may be constructed so as to advantageously horizontally wick fluid, such as wound exudate, as it is absorbed upward through the layers of the dressing 550D. The ADL may comprise cellulose in the range of 40-160 gsm (or about 40 to about 160 gsm), for example 80 (or about 80) gsm.. The ADL may be constructed from a material which resists compression. The ADL material, in an uncompressed state, may be 0.5 mm to 3 mm thick, or approximately 0.5 mm to approximately 3 mm thick, and in some embodiments may be 1.2 mm thick, or approximately 1.2 mm thick, in an uncompressed state. The ADL material may comprise a plurality of loosely packed fibers, which may be arranged in a substantially horizontal fibrous network.

In some aspects, the ADL material may consist of a mix of two fiber types. One may be a flat fiber which may be 20 µm to 50 µm in width, or approximately 20 µm to approximately 50 µm in width, and may comprise a cellulosic based material. The other fiber may be a two component fiber that has an inner core that is 8 µm to 10 µm in diameter, or approximately is 8 µm to approximately 10 µm in diameter, and an outer layer with a thickness of 1 µm to 2 µm, or approximately 1 µm to approximately 2 µm. The two component fiber may be a mix of a polyethylene (PE) type material, and polyethylene terephthalate (PET). In some embodiments the inner core of the two component fiber may be PET and the outer layer may be PE. The PE/PET fibers may have a smooth surface morphology, while the cellulosic fibers may have a relatively rougher surface morphology. In some embodiments the ADL material may comprise about 60% to about 90% cellulosic fibers, for example approximately 75% cellulosic fibers, and may comprise about 10% to about 40% PE/PET fibers, for example approximately 25% PE/PET fibers.

In some aspects, a majority of the fiber volume of the ADL may extend horizontally (that is, parallel to the plane of the top and bottom surfaces of the material), or substantially or generally horizontally. In some variants, 80%-90% (or approximately 80% to approximately 90%) or more of the fiber volume of the ADL may extend horizontally, or substantially or generally horizontally. In another variant, a majority, 80%-90% (or approximately 80% to approximately 90%) of the fibers or more, or even all or substantially all of the fibers, span a distance perpendicular to the thickness of the ADL material (a horizontal or lateral distance) that is greater than the thickness of the ADL material. In some embodiments, the horizontal or lateral distance spanned by such fibers is 2 times (or about 2 times) or more, 3 times (or about 3 times) or more, 4 times (or about 4 times) or more, 5 times (or about 5 times) or more, or 10 times (or about 10 times) or more the thickness of the ADL material. The orientation of such fibers may promote lateral wicking of fluid through the ADL material. This may more evenly distribute fluid such as wound exudate throughout the ADL material.

In some aspects, the fluid handling layer 170 can be or include a spacer or transmission layer. The spacer or transmission material can be preferably formed of a material having a three dimensional structure, and may have a top layer and a bottom layer comprising a knit pattern. For example, a knitted or woven spacer fabric (for example Baltex 7970 weft knitted polyester) or a non-woven fabric could be used. The top and bottom fabric layers may comprise polyester, such as 84/144 textured polyester or a flat denier polyester. Other materials and other linear mass densities of fiber could of course be used. In some aspects, the top and bottom fabric layers may be the same pattern and the same material, and in other aspects they may be different patterns and/or different materials. The top fabric layer may have more filaments in a yarn used to form it than the number of filaments making up the yarn used to form the bottom fabric layer, in order to control moisture flow across the transmission layer. Particularly, by having a filament count greater in the top layer, that is to say, the top layer is made from a yarn having more filaments than the yarn used in the bottom layer, liquid tends to be wicked along the top layer more than the bottom layer.

In some aspects, the spacer or transmission layer can have a majority of the filaments, by volume, extending vertically (that is, perpendicular to the plane of the top and bottom layers), or substantially or generally vertically. In some aspects, 80%-90% (or approximately 80% to approximately 90%) of the filaments or more, by volume, may extend vertically, or substantially or generally vertically. In another variant, all or substantially all of the filaments, by volume, may extend vertically, or substantially or generally vertically. In some aspects, a majority, 80%-90% (or approximately 80% to approximately 90%) of the filaments or more, or even all or substantially all of the filaments, extend upward from the bottom fabric layer and/or downward from the top fabric layer, and in some aspects, such filaments extend over a length more than half the distance between the top and bottom fabric layers. In some aspects, a majority, 80%-90% (or approximately 80% to approximately 90%) of the filaments or more, or even all or substantially all of the filaments, span a distance that is greater in a direction perpendicular to the top and bottom fabric layers (a vertical direction) than in a direction parallel to the top and bottom fabric layers (a horizontal direction). The orientation of such filaments may promote vertical wicking of fluid through the spacer layer. Such filaments may also keep the top and bottom layers spaced apart when the dressing 550D is exposed to compressive forces or negative pressure.

In the illustrated embodiment of Figure 34, the fluid handling layer 170 is disposed between the housing 600D and the film layer 154D. Additionally and alternatively, the fluid handling layer 170 can be disposed at other locations within the dressing 550D. In some aspects, the fluid handling layer 170 can be disposed at or near the skin interface when the dressing 550D is attached to the skin of the wearer of the dressing 550D. For example, the fluid handling layer 170 can be disposed between the contact layer 160D and the adhesive spread layer 158D of the dressing 550D. Additionally and alternatively, the fluid handling layer 170 can be disposed between the release handle 156D and the wound contact layer 160D such that the fluid handling layer 170 is in direct contact with the skin of the wearer of the dressing 550D when the release handle 156D is removed to apply the dressing 550D to the skin of the wearer. Additionally and alternatively, the fluid handling layer 170 can be sandwiched between the contact layer 160D and the adhesive spread layer 158D. The dressing 550D can include fluid handling layers 170 that are different in type. For example, the dressing 550D can have a first fluid handling layer 170 that is a foam layer disposed between the contact layer 160D and the adhesive spread layer 158D, and a second fluid handling layer 170 that is an ADL disposed between the housing 600D and the film layer 154D. All combinations of the possible aforementioned arrangements of the fluid handling layer 170 in the dressing 550D are within the scope of the present disclosure and are omitted for the sake of clarity.

**Figure 35** illustrates an embodiment of a housing 600E and a dressing 550E and shows an approach for coupling the housing 600E to the dressing 550E. The illustrated housing 600E includes a plurality of grooves or channels 606, as discussed. In some variants, the housing 600E does not include any grooves or channels 606. The dressing 550E can include a fluid handling layer 170 disposed between a film layer 154E and a contact layer 160E. In some aspects, the fluid handing layer 170 is foam. In some variants, the dressing 550E can be an ALLEVYN^{™} dressing sold by Smith & Nephew (e.g., an ALLEVYN^{™} LIFE dressing) or any other suitable dressing.

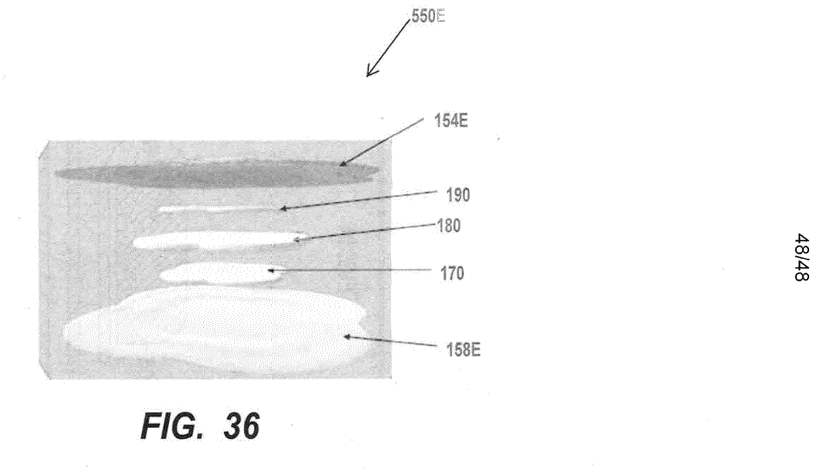

**Figure 36** shows a non-limiting, illustrative embodiment of the dressing 550E. The dressing 550E can have multiple layers and the multiple layers can be arranged other than shown in the illustrative embodiment depicted in Figure 36. For example, the dressing 550E can include a film layer 154E and an optional bottom wound contact layer 160E (not shown in Figure 36) attached to a bottom surface of an adhesive spread layer 158E (e.g. a silicone adhesive layer). In some variants, the film layer 154E may be permeable to moisture and/or air. The dressing 550E can include various layers positioned between the wound contact layer 160E and the film layer 154E. For example, the dressing 550E can include one or more absorbent layers 180 or one or more fluid handling layers 170. In some aspects, the dressing 550E can include a perforated wound contact layer 160E and a top film layer 154E. In the illustrated embodiment, the wound contact layer 160E is not shown because it is obscured by the adhesive spread layer 158E that directly overlays the wound contact layer 160E. Further components of the dressing 550E can include a fluid handling layer 170 that is a foam layer, such as a layer of polyurethane hydrocellular foam, of a suitable size to cover the recommended dimension of wounds corresponding to the particular dressing size chosen. An optional layer of activated charcoal cloth (not shown) of similar or slightly smaller dimensions than the foam layer may be provided to allow for odor control. An absorbent layer 180, such as a layer of superabsorbent air-laid material containing cellulose fibers and a superabsorbent polyacrylate particulates, can be provided over the foam layer 170, of dimensions slightly larger than the foam layer, and can allow for an overlap of superabsorbent material and can act as leak prevention. A masking or obscuring layer 190, such as a layer of three-dimensional knitted spacer fabric, can be provided over the absorbent layer 180, providing protection from pressure, while allowing partial masking of the top surface of the superabsorber where colored exudate would remain. The smaller dimension (in plan view) of the masking layer 190 compared to the absorbent layer 180 can allow for visibility of the edge of the absorbent layer 180, which can be used by clinicians to assess whether the dressing needs to be changed.

The housing 600E can be attached to the outer surface of the film layer 154E such that the film layer 154E is sandwiched between the housing 600E and the masking layer 190. In some aspects, placing the housing 600E on top of a foam-containing dressing 550E can reduce the likelihood of the housing 600E being knocked off of the dressing 550E by an impact to the housing 600E. As shown in Figure 35, the housing 600E can be adhered to the outer surface of the film layer 154E. In this position, as the housing 600E is impacted or moved by external forces, the foam (e.g., the fluid handling layer 170) will allow the housing 600E to move, thereby reducing the forces applied to the skin and/or the adhesive spread layer 158E of the dressing 550E. In some aspects, the foam can help the contact layer 160E to conform to the curvature of the wearer's body shape. The foam can compress as required to allow the rigid, flat base of the housing 600E to sit more comfortably on top of the skin of the wearer.

Turning back to Figure 35, the dressing 550E can optionally include a cushion layer 172 disposed under the housing 600E. In some variants, the cushion layer 172 can be an OPSITE^{™} Post-Op Visible dressing or portion thereof sold by Smith & Nephew or any other suitable dressing. For example, the cushion layer 172 can comprise foam having openings or slits therein. Further details of the cushion layer 172 are found in International Application No. PCT/US2007/079529, filed on September 26, 2006, entitled "LATTICE DRESSING," published on April 3, 2008 as International Publication No. WO 2008/039839, and incorporated in its entirety by reference herein. In some variants, the cushion layer 172 can be a contact layer 160 (e.g., a silicone wound contact layer 160). The cushion layer 172 can cushion the area under the housing 600E and provide the ability for moisture to move under and around the housing 600E. In some variants, the dressing 550E does not include a cushion layer 172. For example, the housing 600E can be adhered directly to the outer surface of the film layer 154E of an ALLEVYN^{™} dressing.

As discussed, the housing 600E can be adhered to the dressing 550E using an adhesive (e.g., an ultraviolet-curable adhesive, an ostomy adhesive, a hydrocolloid adhesive), as described herein. In some variants, an ultraviolet-curable adhesive is applied to the bottom surface of the housing 600E which is then placed on the outer surface of the film layer 154E to form an assembly. The assembly can be placed onto a curing shelf 500 (shown in Figure 22C) in a first position such that the dressing 550E is disposed between the housing 600E and the curing shelf 500. The curing process can be set to last about 30 seconds with the assembly on the curing shelf 500 in the first position. In some aspects, the assembly of the housing 600E and the dressing 550E can placed on the curing shelf 500 in a second position so that the housing 600E is in direct contact with the curing shelf 500 and is disposed between the curing shelf 500 and the dressing 550E. In some aspects, the curing process can be performed for about 60 seconds with the assembly on the curing shelf 500 in the second position. In some variants, the method includes performing the curing procedure with the assembly in the first position for 30 seconds, moving the assembly into the second position, and performing the curing procedure with the assembly in the second position for 60 seconds.

### Example Implementations

An activity-monitoring device for positioning on a body of a user or on an orthopedic device is disclosed. The activity-monitoring device can include: a housing including a cap portion and a base portion, wherein the cap portion includes an inner rim that extends from an upper wall of the cap portion, a recessed portion disposed on a base-facing surface of the upper wall, a boss extending from the recessed portion, wherein the base portion includes an outer sidewall that extends from a cap-facing surface of the base portion, the inner rim and the outer sidewall can connect with one another to form an enclosed space therebetween, a thickness of the upper wall at the recessed portion being thinner than a thickness of the upper wall outside of the recessed portion; a circuit board assembly positioned in the cap portion, wherein the circuit board assembly includes a controller and a button, the button can be pressed by the boss to activate the controller; and a power source positioned in the cap portion and configured to power the controller.

The activity-monitoring device of the preceding paragraph can include one or more of the following features: The activity-monitoring device can further include a sensor, which is a gyroscope, a magnetometer, an accelerometer, a barometer, a light sensor, and a temperature sensor. The sensor can detect a movement of the housing. The controller can monitor the movement of the housing using the sensor. The controller can store the movement of the housing to a memory device and output the movement of the housing to an electronic device via a communication interface. The activity-monitoring device can further include a light source disposed on the circuit board assembly, the light source being arranged to illuminate through the recessed portion when the light source is activated. The cap portion can include one or more pins for aligning the circuit board assembly with the cap portion. The activity-monitoring device can include a dressing including an upper surface and a lower surface, wherein the base portion is configured to be adhered to the upper surface. The activity-monitoring device can include an orthopedic device, wherein the base portion is configured to be adhered to the orthopedic device.

### Other Variations and Terminology

Any value of a threshold, limit, duration, etc. provided herein is not intended to be absolute and, thereby, can be approximate. In addition, any threshold, limit, duration, etc. provided herein can be fixed or varied either automatically or by a user. Furthermore, as is used herein relative terminology such as exceeds, greater than, less than, etc. in relation to a reference value is intended to also encompass being equal to the reference value. For example, exceeding a reference value that is positive can encompass being equal to or greater than the reference value. In addition, as is used herein relative terminology such as exceeds, greater than, less than, etc. in relation to a reference value is intended to also encompass an inverse of the disclosed relationship, such as below, less than, greater than, etc. in relations to the reference value. Moreover, although blocks of the various processes maybe described in terms of determining whether a value meets or does not meet a particular threshold, the blocks can be similarly understood, for example, in terms of a value (i) being below or above a threshold or (ii) satisfying or not satisfying a threshold.

Features, materials, characteristics, or groups described in conjunction with a particular aspect, embodiment, or example are to be understood to be applicable to any other aspect, embodiment or example described herein unless incompatible therewith. All of the features disclosed in this specification (including any accompanying claims, abstract and drawings), or all of the steps of any method or process so disclosed, may be combined in any combination, except combinations where at least some of such features or steps are mutually exclusive. The protection is not restricted to the details of any foregoing embodiments. The protection extends to any novel one, or any novel combination, of the features disclosed in this specification (including any accompanying claims, abstract and drawings), or to any novel one, or any novel combination, of the steps of any method or process so disclosed.

While certain embodiments have been described, these embodiments have been presented by way of example only, and are not intended to limit the scope of protection. Indeed, the novel methods and systems described herein maybe embodied in a variety of other forms. For example, while the housing has been shown to be mounted on an outer surface of the dressing, in some variants the housing and/or the electronics of the activity-monitoring device can be encapsulated in the dressing. Furthermore, various omissions, substitutions and changes in the form of the methods and systems described herein may be made. Those skilled in the art will appreciate that in some embodiments, the actual steps taken in the processes illustrated or disclosed may differ from those shown in the figures. Depending on the embodiment, certain of the steps described above may be removed, others may be added. For example, the actual steps or order of steps taken in the disclosed processes may differ from those shown in the figure. Depending on the embodiment, certain of the steps described above may be removed, others may be added. For instance, the various components illustrated in the figures may be implemented as software or firmware on a processor, controller, ASIC, FPGA, or dedicated hardware. Hardware components, such as controllers, processors, ASICs, FPGAs, and the like, can include logic circuitry. Furthermore, the features and attributes of the specific embodiments disclosed above may be combined in different ways to form additional embodiments, all of which fall within the scope of the present disclosure.

Although the present disclosure includes certain embodiments, examples and applications, it will be understood by those skilled in the art that the present disclosure extends beyond the specifically disclosed embodiments to other alternative embodiments or uses and obvious modifications and equivalents thereof, including embodiments which do not provide all of the features and advantages set forth herein. Accordingly, the scope of the present disclosure is not intended to be limited by the specific disclosures of preferred embodiments herein, and may be defined by claims as presented herein.

Conditional language, such as "can," "could," "might," or "may," unless specifically stated otherwise, or otherwise understood within the context as used, is generally intended to convey that certain embodiments include, while other embodiments do not include, certain features, elements, or steps. Thus, such conditional language is not generally intended to imply that features, elements, or steps are in any way required for one or more embodiments or that one or more embodiments necessarily include logic for deciding, with or without user input or prompting, whether these features, elements, or steps are included or are to be performed in any particular embodiment. The terms "comprising," "including," "having," and the like are synonymous and are used inclusively, in an open-ended fashion, and do not exclude additional elements, features, acts, operations, and so forth. Also, the term "or" is used in its inclusive sense (and not in its exclusive sense) so that when used, for example, to connect a list of elements, the term "or" means one, some, or all of the elements in the list. Further, the term "each," as used herein, in addition to having its ordinary meaning, can mean any subset of a set of elements to which the term "each" is applied.

Conjunctive language such as the phrase "at least one of X, Y, and Z," unless specifically stated otherwise, is otherwise understood with the context as used in general to convey that an item, term, etc. may be either X, Y, or Z. Thus, such conjunctive language is not generally intended to imply that certain embodiments require the presence of at least one of X, at least one of Y, and at least one of Z.

Language of degree used herein, such as the terms "approximately," "about," "generally," and "substantially" as used herein represent a value, amount, or characteristic close to the stated value, amount, or characteristic that still performs a desired function or achieves a desired result. For example, the terms "approximately", "about", "generally," and "substantially" may refer to an amount that is within less than 10% of, within less than 5% of, within less than 1% of, within less than 0.1% of, and within less than 0.01% of the stated amount. As another example, in certain embodiments, the terms "generally parallel" and "substantially parallel" refer to a value, amount, or characteristic that departs from exactly parallel by less than or equal to 15 degrees, 10 degrees, 5 degrees, 3 degrees, 1 degree, or 0.1 degree.

The scope of the present disclosure is not intended to be limited by the specific disclosures ofpreferred embodiments in this section or elsewhere in this specification, and the scope of the invention is defined in claim 1. The language of the claims is to be interpreted broadly based on the language employed in the claims and not limited to the examples described in the present specification or during the prosecution of the application, which examples are to be construed as non-exclusive.

## Claims

1. An activity monitoring device (120) for positioning on a body of a user or on an orthopedic device, the activity monitoring device comprising:
a housing (400);
a circuit board assembly positioned in the housing (400), the circuit board assembly comprising a sensor (210) and a controller (202), the sensor (210) being configured to detect a movement of the circuit board assembly, the controller (202) being configured to monitor the movement of the circuit board assembly with the sensor (210); and
a dressing (150) having an upper surface and a lower surface, the upper surface being adhered to or configured to be adhered to the housing (400), the lower surface being configured to attach to the user or to the orthopedic device, the dressing comprising a flexible film layer (154),
wherein the flexible film layer (154) defines at least in part the upper surface of the dressing (150), and wherein the flexible film layer (154) is adhered to a base portion (404) of the housing (400) by an adhesive.

2. The activity monitoring device of claim 1, wherein the dressing further comprises a spacer layer (164).

3. The activity monitoring device (120) of any one of the preceding claims, wherein the dressing comprises a film dressing layer (162) configured to secure the housing to the flexible film layer (154).

4. The activity monitoring device (120) of claim 1, wherein the film dressing layer (162) configured to secure the housing (400) and the flexible film layer (154) to the user or the orthopedic device.

5. The activity device (120) of claim 2 or 3, wherein the film dressing layer (162) is vacuumed over the housing (400).

6. The activity monitoring device (120) of any one of the preceding claims, wherein the flexible film layer (154) comprises polyurethane or polyethylene; optionally
wherein the flexible film layer (154) is moisture vapor permeable.

7. The activity monitoring device (120) of any one of the preceding claims, wherein the dressing comprises a contact layer defining at least in part the lower surface of the dressing, the contact layer being configured to attach to the user or to the orthopedic device; optionally
wherein the contact layer (160) comprises a silicone adhesive defining at least in part the lower surface of the dressing.

8. The activity monitoring device (120) of claim 7, wherein the contact layer (160) is perforated.

9. The activity monitoring device (120) of claim 7 or claim 8, wherein the contact layer (160) is moisture vapor permeable.

10. The activity monitoring device (120)of any one of claims 7 to 9, wherein the contact layer (160) is positioned underneath the film layer (154) in a location underlying the housing (400) and has an area at least as large as a lower surface of the housing (400), the contact layer (160) being surrounded by a lower surface of the flexible film layer.

11. The activity monitoring device (120) of any one of the preceding claims, wherein the housing (400) comprises a top surface and a base surface, the base surface being adhered to or configured to be adhered the upper surface, an outer surface of the housing (400) flaring out proximate to the base surface.

12. The activity monitoring device (120) of any one of the preceding claims, wherein the housing (400) comprises a base portion (404) and a cap portion (402), the base portion (404) comprising an outer sidewall (408) that extends from a cap-facing surface of the base portion (404), the outer sidewall (408) configured to connect with an inner rim (406) extending from an upper wall of the cap portion (402) to form an enclosed space therebetween, a first recessed portion (414) disposed on a base-facing surface of the upper wall, a second recessed portion disposed on the first recessed portion (414).

13. The activity monitoring device (120) of any one of the preceding claims, wherein the dressing (150) comprises a frame delivery layer (152) positioned over the film layer (154).

14. The activity monitoring device (120) of any one of the preceding claims, wherein the dressing (150) comprises a release handle (156) positioned below the film layer (154).

15. The activity monitoring device (120) of any one of the preceding claims, wherein the sensor (210) is selected from the group consisting of: a gyroscope, a magnetometer, an accelerometer, a barometer, a light sensor, and a temperature sensor.

16. The activity monitoring device (120) of any one of the preceding claims, wherein the controller (202) is configured to store the movement to a memory device and output the movement of the housing (400) to an electronic device via a communication interface.

## Patentansprüche

1. Eine Aktivitätsüberwachungsvorrichtung (120) zur Positionierung an einem Körper eines Benutzers oder an einer orthopädischen Vorrichtung, wobei die Aktivitätsüberwachungsvorrichtung Folgendes beinhaltet:
ein Gehäuse (400);
eine in dem Gehäuse (400) positionierte Leiterplattenbaugruppe, wobei die Leiterplattenbaugruppe einen Sensor (210) und eine Steuerung (202) beinhaltet, wobei der Sensor (210) konfiguriert ist, um eine Bewegung der Leiterplattenbaugruppe zu erkennen, wobei die Steuerung (202) konfiguriert ist, um die Bewegung der Leiterplattenbaugruppe mit dem Sensor (210) zu überwachen; und
einen Verband (150), der eine obere Oberfläche und eine untere Oberfläche aufweist, wobei die obere Oberfläche an dem Gehäuse (400) klebt oder konfiguriert ist, um daran zu kleben, wobei die untere Oberfläche konfiguriert ist, um an dem Benutzer oder an der orthopädischen Vorrichtung befestigt zu sein, wobei der Verband eine flexible Folienschicht (154) beinhaltet,
wobei die flexible Folienschicht (154) mindestens teilweise die obere Oberfläche des Verbands (150) definiert und wobei die flexible Folienschicht (154) mit einem Kleber an einem Basisabschnitt (404) des Gehäuses (400) klebt.

2. Aktivitätsüberwachungsvorrichtung gemäß Anspruch 1, wobei der Verband ferner eine Abstandshalterschicht (164) beinhaltet.

3. Aktivitätsüberwachungsvorrichtung (120) gemäß einem der vorhergehenden Ansprüche, wobei der Verband eine Folienverbandschicht (162) beinhaltet, die konfiguriert ist, um das Gehäuse an der flexiblen Folienschicht (154) zu sichern.

4. Aktivitätsüberwachungsvorrichtung (120) gemäß Anspruch 1, wobei die Folienverbandschicht (162) konfiguriert ist, um das Gehäuse (400) und die flexible Folienschicht (154) an dem Benutzer oder der orthopädischen Vorrichtung zu sichern.

5. Aktivitätsvorrichtung (120) gemäß Anspruch 2 oder 3, wobei die Folienverbandschicht (162) mittels Unterdruck über das Gehäuse (400) gezogen ist.

6. Aktivitätsüberwachungsvorrichtung (120) gemäß einem der vorhergehenden Ansprüche, wobei die flexible Folienschicht (154) Polyurethan oder Polyethylen beinhaltet; wobei optional
die flexible Folienschicht (154) wasserdampfdurchlässig ist.

7. Aktivitätsüberwachungsvorrichtung (120) gemäß einem der vorhergehenden Ansprüche, wobei der Verband eine Kontaktschicht beinhaltet, die mindestens teilweise die untere Oberfläche des Verbands definiert, wobei die Kontaktschicht konfiguriert ist, um an dem Benutzer oder an der orthopädischen Vorrichtung befestigt zu sein; wobei optional
die Kontaktschicht (160) einen Silikonkleber beinhaltet, der mindestens teilweise die untere Oberfläche des Verbands definiert.

8. Aktivitätsüberwachungsvorrichtung (120) gemäß Anspruch 7, wobei die Kontaktschicht (160) perforiert ist.

9. Aktivitätsüberwachungsvorrichtung (120) gemäß Anspruch 7 oder Anspruch 8, wobei die Kontaktschicht (160) wasserdampfdurchlässig ist.

10. Aktivitätsüberwachungsvorrichtung (120) gemäß einem der Ansprüche 7 bis 9, wobei die Kontaktschicht (160) unter der Folienschicht (154) an einer Stelle, die unter dem Gehäuse (400) liegt, positioniert ist und eine Fläche aufweist, die mindestens so groß ist wie eine untere Oberfläche des Gehäuses (400), wobei die Kontaktschicht (160) von einer unteren Oberfläche der flexiblen Folienschicht umgeben ist.

11. Aktivitätsüberwachungsvorrichtung (120) gemäß einem der vorhergehenden Ansprüche, wobei das Gehäuse (400) eine obere Oberfläche und eine Basisoberfläche beinhaltet, wobei die Basisoberfläche an der oberen Oberfläche klebt oder konfiguriert ist, um daran zu kleben, wobei sich eine äußere Oberfläche des Gehäuses (400) in der Nähe der Basisoberfläche ausweitet.

12. Aktivitätsüberwachungsvorrichtung (120) gemäß einem der vorhergehenden Ansprüche, wobei das Gehäuse (400) einen Basisabschnitt (404) und einen Kappenabschnitt (402) beinhaltet, wobei der Basisabschnitt (404) eine äußere Seitenwand (408) beinhaltet, die sich von einer der Kappe zugewandten Oberfläche des Basisabschnitts (404) erstreckt, wobei die äußere Seitenwand (408) konfiguriert ist, um sich einem inneren Rand (406), der sich von einer oberen Wand des Kappenabschnitts (402) erstreckt, zu verbinden, um dazwischen einen geschlossenen Raum zu bilden, wobei ein erster ausgesparter Abschnitt (414) auf einer der Basis zugewandten Oberfläche der oberen Wand angeordnet ist, wobei ein zweiter ausgesparter Abschnitt auf dem ersten ausgesparten Abschnitt (414) angeordnet ist.

13. Aktivitätsüberwachungsvorrichtung (120) gemäß einem der vorhergehenden Ansprüche, wobei der Verband (150) eine über der Folienschicht (154) positionierte Rahmenabgabeschicht (152) beinhaltet.

14. Aktivitätsüberwachungsvorrichtung (120) gemäß einem der vorhergehenden Ansprüche, wobei der Verband (150) ein unterhalb der Folienschicht (154) positioniertes Freigabegriffstück (156) beinhaltet.

15. Aktivitätsüberwachungsvorrichtung (120) gemäß einem der vorhergehenden Ansprüche, wobei der Sensor (210) aus der Gruppe ausgewählt ist, die aus Folgendem besteht: einem Gyroskop, einem Magnetometer, einem Beschleunigungsmesser, einem Barometer, einem Lichtsensor und einem Temperatursensor.

16. Aktivitätsüberwachungsvorrichtung (120) gemäß einem der vorhergehenden Ansprüche, wobei die Steuerung (202) konfiguriert ist, um die Bewegung in einer Speichervorrichtung zu speichern und die Bewegung des Gehäuses (400) über eine Kommunikationsschnittstelle an eine elektronische Vorrichtung auszugeben.

## Revendications

1. Un dispositif de surveillance d'activité (120) destiné à être positionné sur un corps d'un utilisateur ou sur un dispositif orthopédique, le dispositif de surveillance d'activité comprenant :
un boîtier (400) ;
un ensemble carte de circuit imprimé positionné dans le boîtier (400), l'ensemble carte de circuit imprimé comprenant un capteur (210) et une unité de commande (202), le capteur (210) étant configuré pour détecter un mouvement de l'ensemble carte de circuit imprimé, l'unité de commande (202) étant configurée pour surveiller le mouvement de l'ensemble carte de circuit imprimé avec le capteur (210) ; et
un pansement (150) ayant une surface supérieure et une surface inférieure, la surface supérieure étant amenée à adhérer au boîtier (400) ou configurée pour être amenée à adhérer à celui-ci, la surface inférieure étant configurée pour s'attacher à l'utilisateur ou au dispositif orthopédique, le pansement comprenant une couche de film souple (154), dans lequel la couche de film souple (154) définit au moins en partie la surface supérieure du pansement (150), et dans lequel la couche de film souple (154) est amenée à adhérer à une portion formant base (404) du boîtier (400) par un adhésif.

2. Le dispositif de surveillance d'activité de la revendication 1, dans lequel le pansement comprend en outre une couche d'espacement (164).

3. Le dispositif de surveillance d'activité (120) de n'importe laquelle des revendications précédentes, dans lequel le pansement comprend une couche de pansement formant film (162) configurée pour assujettir le boîtier à la couche de film souple (154).

4. Le dispositif de surveillance d'activité (120) de la revendication 1, dans lequel la couche de pansement formant film (162) est configurée pour assujettir le boîtier (400) et la couche de film souple (154) à l'utilisateur ou au dispositif orthopédique.

5. Le dispositif d'activité (120) de la revendication 2 ou de la revendication 3, dans lequel la couche de pansement formant film (162) est disposée sous vide par-dessus le boîtier (400).

6. Le dispositif de surveillance d'activité (120) de n'importe laquelle des revendications précédentes, dans lequel la couche de film souple (154) comprend du polyuréthane ou du polyéthylène ; facultativement
dans lequel la couche de film souple (154) est perméable à la vapeur d'eau.

7. Le dispositif de surveillance d'activité (120) de n'importe laquelle des revendications précédentes, dans lequel le pansement comprend une couche de contact définissant au moins en partie la surface inférieure du pansement, la couche de contact étant configurée pour s'attacher à l'utilisateur ou au dispositif orthopédique ; facultativement dans lequel la couche de contact (160) comprend un adhésif en silicone définissant au moins en partie la surface inférieure du pansement.

8. Le dispositif de surveillance d'activité (120) de la revendication 7, dans lequel la couche de contact (160) est perforée.

9. Le dispositif de surveillance d'activité (120) de la revendication 7 ou de la revendication 8, dans lequel la couche de contact (160) est perméable à la vapeur d'eau.

10. Le dispositif de surveillance d'activité (120) de n'importe laquelle des revendications 7 à 9, dans lequel la couche de contact (160) est positionnée sous la couche de film (154) en un emplacement sous-jacent au boîtier (400) et a une aire au moins aussi grande qu'une surface inférieure du boîtier (400), la couche de contact (160) étant entourée d'une surface inférieure de la couche de film souple.

11. Le dispositif de surveillance d'activité (120) de n'importe laquelle des revendications précédentes, dans lequel le boîtier (400) comprend une surface de dessus et une surface de base, la surface de base étant amenée à adhérer à la surface supérieure ou configurée pour être amenée à adhérer à celle-ci, une surface externe du boîtier (400) s'évasant à proximité de la surface de base.

12. Le dispositif de surveillance d'activité (120) de n'importe laquelle des revendications précédentes, dans lequel le boîtier (400) comprend une portion formant base (404) et une portion formant coiffe (402), la portion formant base (404) comprenant une paroi latérale externe (408) qui s'étend à partir d'une surface faisant face à la coiffe de la portion formant base (404), la paroi latérale externe (408) étant configurée pour se raccorder à un rebord interne (406) s'étendant à partir d'une paroi supérieure de la portion formant coiffe (402) afin de former un espace clos entre eux, une première portion en renfoncement (414) disposée sur une surface faisant face à la base de la paroi supérieure, une deuxième portion en renfoncement disposée sur la première portion en renfoncement (414).

13. Le dispositif de surveillance d'activité (120) de n'importe laquelle des revendications précédentes, dans lequel le pansement (150) comprend une couche d'apport formant cadre (152) positionnée par-dessus la couche de film (154).

14. Le dispositif de surveillance d'activité (120) de n'importe laquelle des revendications précédentes, dans lequel le pansement (150) comprend un élément de préhension anticollant (156) positionné en dessous de la couche de film (154).

15. Le dispositif de surveillance d'activité (120) de n'importe laquelle des revendications précédentes, dans lequel le capteur (210) est sélectionné dans le groupe constitué : d'un gyroscope, d'un magnétomètre, d'un accéléromètre, d'un baromètre, d'un capteur de luminosité, et d'un capteur de température.

16. Le dispositif de surveillance d'activité (120) de n'importe laquelle des revendications précédentes, dans lequel l'unité de commande (202) est configurée pour stocker le mouvement sur un dispositif mémoire et fournir en sortie le mouvement du boîtier (400) à un dispositif électronique par l'intermédiaire d'une interface de communication.
